(19) Europäisches Patentamt / European Patent Office / Office européen des brevets

(11) **EP 4 401 799 B1**

(12) **EUROPEAN PATENT SPECIFICATION**

(45) Date of publication and mention of the grant of the patent:
**01.07.2026 Bulletin 2026/27**

(21) Application number: **22789191.8**

(22) Date of filing: **14.09.2022**

(51) International Patent Classification (IPC):
*A61L 15/28* (2006.01)     *A61L 15/40* (2006.01)
*A61L 15/42* (2006.01)     *A61L 24/00* (2006.01)
*A61L 24/08* (2006.01)     *A61L 24/10* (2006.01)

(52) Cooperative Patent Classification (CPC):
(C-Sets available)
**A61L 15/28; A61L 15/40; A61L 15/425;
A61L 24/0036; A61L 24/08; A61L 24/10;**
A61L 2400/04                                              (Cont.)

(86) International application number:
**PCT/EP2022/075521**

(87) International publication number:
**WO 2023/041578 (23.03.2023 Gazette 2023/12)**

(54) **SPONGE-LIKE SCAFFOLD FOR PROMOTING HAEMOSTASIS**

SCHWAMMARTIGES GERÜST ZUR FÖRDERUNG DER HÄMOSTASE

ÉCHAFAUDAGE SPONGIEUX POUR FAVORISER L'HÉMOSTASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.09.2021 EP 21382834**

(43) Date of publication of application:
**24.07.2024 Bulletin 2024/30**

(73) Proprietor: **Universidad del País Vasco/Euskal Herriko Unibertsitatea
48940 Leioa, Vizcaya (ES)**

(72) Inventors:
• **HERNÁNDEZ MARTÍN, Rosa María**
  **48940 Leioa, Vizcaya (ES)**
• **SANTOS VIZCAÍNO, Edorta**
  **48940 Leioa, Vizcaya (ES)**
• **IGARTUA OLAECHEA, Manuela**
  **48940 Leioa, Vizcaya (ES)**
• **LAS HERAS ZAPATA, Kevin**
  **48940 Leioa, Vizcaya (ES)**
• **JIMÉNEZ MARTÍN, Jon**
  **48940 Leioa, Vizcaya (ES)**
• **DE LA CABA CIRIZA, María Coro**
  **48940 Leioa, Vizcaya (ES)**
• **GUERRERO MANSO, Pedro Manuel**
  **48940 Leioa, Vizcaya (ES)**
• **ETXABIDE ETXEBERRIA, Alaitz**
  **48940 Leioa, Vizcaya (ES)**

(74) Representative: **ABG Intellectual Property Law, S.L.
Avenida de Burgos, 16D
Edificio Euromor
28036 Madrid (ES)**

(56) References cited:
**WO-A1-2017/015488**

• **M MINCEA ET AL: "PREPARATION, MODIFICATION, AND APPLICATIONS OF CHITIN NANOWHISKERS: A REVIEW", REV. ADV. MATER. SCI, 1 January 2012 (2012-01-01), pages 225 - 242, XP055310175, Retrieved from the Internet <URL:https://www.ipme.ru/e-journals/RAMS/no_33012/02_mincea.pdf>**

EP 4 401 799 B1

• SONG XIAOQIANG ET AL: "Effects of degree of deacetylation on hemostatic performance of partially deacetylated chitin sponges", CARBOHYDRATE POLYMERS, APPLIED SCIENCE PUBLISHERS , LTD BARKING, GB, vol. 273, 28 August 2021 (2021-08-28), XP086792327, ISSN: 0144-8617, [retrieved on 20210828], DOI: 10.1016/J.CARBPOL.2021.118615

(52) Cooperative Patent Classification (CPC): (Cont.)

C-Sets
A61L 15/28, C08L 5/08;
A61L 24/08, C08L 5/08

## Description

### TECHNICAL FIELD OF THE INVENTION

The present invention relates to a sponge-like scaffold for use in promoting haemostasis and the treatment of bleeding wounds. Therefore, the field of the invention can be considered to be medicine, specifically preparations for medical purposes.

### BACKGROUND OF THE INVENTION

Haemostasis is the process to prevent and stop bleeding. Haemostasis occurs when the blood is present outside the body or blood vessels. It is the innate response for the body to stop bleeding and loss of blood. During haemostasis three steps occur in a rapid sequence: vascular spasm is the first step wherein the blood vessels constrict to allow less blood to be lost; platelet plug formation is the second step, where platelets stick together to form a temporary seal to cover the break in the vessel wall; and the final third step is coagulation or blood clothing, which reinforces the platelet plug with fibrin threads that act like a "molecular glue". Hemostasis can be achieved in various other ways if the body cannot do it naturally (or needs help) during surgery or medical treatment. When the body is under shock and stress, hemostasis is harder to achieve.

An example of a haemostasis process occurs during a nasal hemorrhage, also known as epistaxis or nosebleed, is one of the most common otorhinolaryngology emergencies worldwide. It is estimated that 60% of the world's population will experience an epistaxis episode at least once in their lifetime, although only about 6% to 10% of them would seek medical attention. Despite being usually simple to treat, severe nosebleed might cause serious risks for the patient, especially for those over 70 years, for whom the incidence of these episodes is higher. In addition to this, nasal injury after nasal surgery is an adverse consequence, for which treatment must be started quickly. Several methods are available to treat epistaxis, among which electrocautery, vasoconstrictors, surgical procedures and nasal packing arise as the most popular.

Materials suitable for promoting haemostasis have been described. For instance, Lu et al (Biomacromolecules 2004, 5:1046) have described hot-pressed nanocomposites films of $\alpha$-chitin nanowhiskers. These compositions are produced through a series of harsh conditions which modify the structure of the chitin. These films, although capable of promoting haemostasis are not ideal as nasal packing devices. Other materials with haemostatic properties include unstructured peptides obtained by treatment of soy protein (WO 2017/015488 A1) or chitosan (deacetylated chitin) (Song et al., 2021, Carbohydrate Polymers, Polymers, Applied Science Publishers, LTD Barking, vol. 73). ()

Although these materials have haemostasic properties, they are not ideal for the treatment of nasal hemorrhage, or for sanitary packing.

Nasal packing, which involves the insertion of a tampon-like product in the nasal cavity, is of special interest for the treatment of epistaxis, offering elevated rates of success. Nevertheless, the choice of the most adequate nasal pack is vital for the outcome of the treatment. The most widely used nasal pack product is a basic gauze while the nasal pack used worldwide as gold standard is Merocel® (MRC), a compressed, dehydrated sponge composed of hydroxylated polyvinyl acetate, being the primary nonabsorbable nasal pack in several emergency departments around the world.

While the ideal nasal pack should promote hemostasis while being comfortable for the patient, as well as hindering mucosal damage, both the gauze and MRC materials are nonabsorbable - causing patient's discomfort during removal - and act just by physical pressure tamponade, without any intrinsic hemostatic effect. Therefore, alternatives to currently commercialized nasal packs are needed.

SONG XIAOQIANG ET AL: "Effects of degree of deacetylation on haemostatic performance of partially deacetylated chitin sponges", CARBOHYDRATE POLYMERS, APPLIED SCIENCE PUBLISHERS, LTD BARKING, GB, vol. 273, 28 August 2021, ISSN: 0144-8617, DOI:10.1016/ J.CARBPOL.2021.11865 discloses the haemostatic properties of chitin sponges.

### SUMMARY OF THE INVENTION

The inventors of the present invention have developed a sponge-like scaffold comprising soy protein, chitin and a plasticizer which has ideal physiochemical and mechanical properties to be used as a haemostasis promoter. Therefore, a first aspect relates to a sponge-like scaffold comprising soy protein (SPI), chitin (CH) and a plasticizer for use in promoting haemostasis.

Another aspect of the present invention relates to a haemostatic product that comprises a sponge-like scaffold as defined in the previous aspect of the present invention.

### BRIEF DESCRIPTION OF THE FIGURES

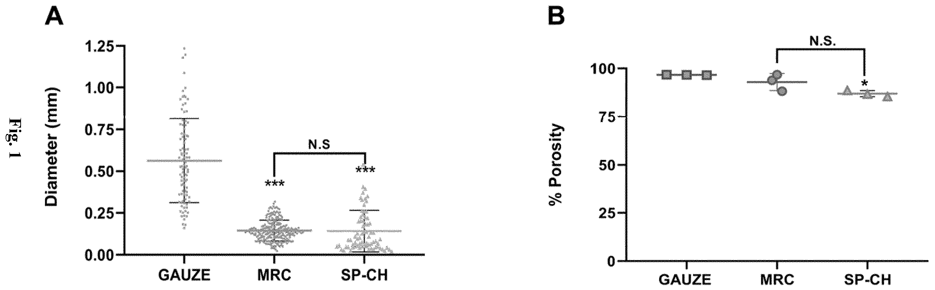

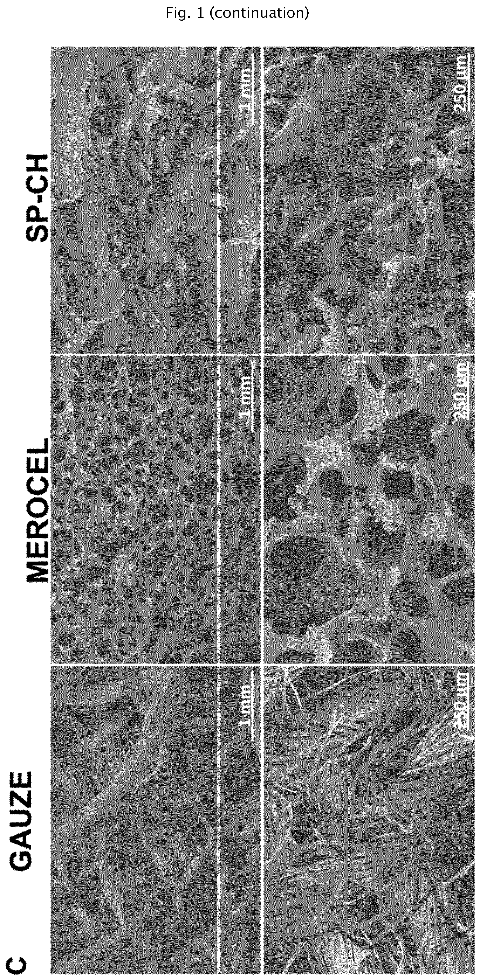

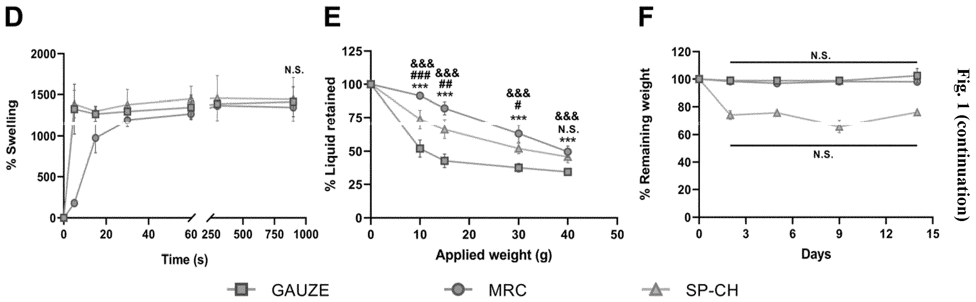

**Figure 1.** Physicochemical characterization. (A) Pore size distribution of the materials. (B) Porosity (%) of each material. Error bars, mean $\pm$ SD. * indicates $p < 0.05$; *** indicates $p < 0.001$ in comparison with the gauze group. N.S., nonsignificant ($p > 0.05$). (C) SEM micrographs of dry materials. (D) Swelling profiles. Error bars, mean $\pm$ SD. N.S., nonsignificant ($p > 0.05$). (E) Liquid retention ratio under different pressures. Error bars, mean $\pm$ SD. &&& indicates $p < 0.001$ by comparing between the gauze and MRC groups. # indicates $p < 0.05$; ##

indicates p < 0.01; ### indicates p < 0.001 by comparing between MRC and SP-CH groups. *** indicates p < 0.001 in by comparing between the gauze and SP-CH groups. (F) Hydrolytic degradation. Error bars, mean $\pm$ SD. N.S., nonsignificant (p > 0.05).

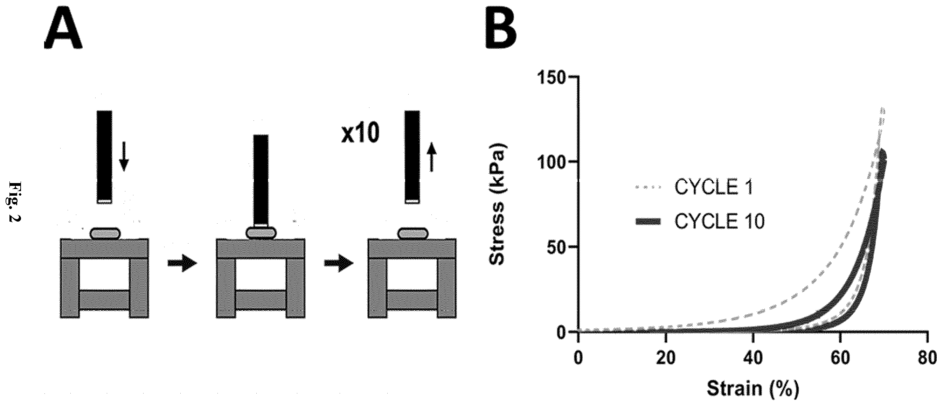

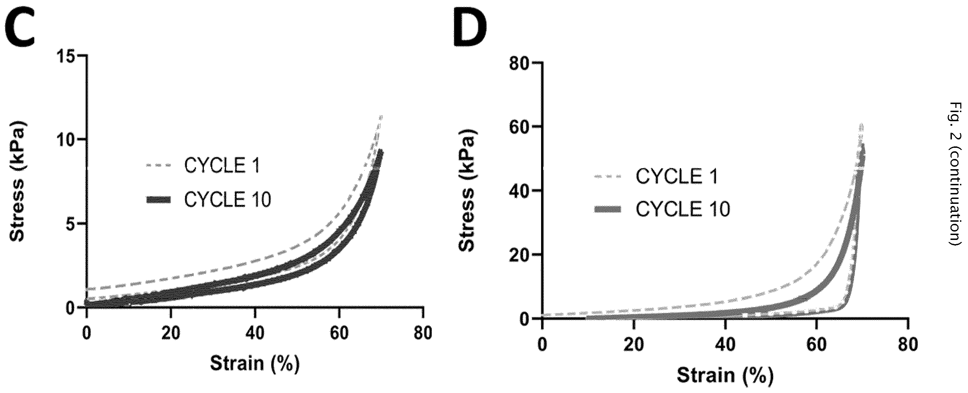

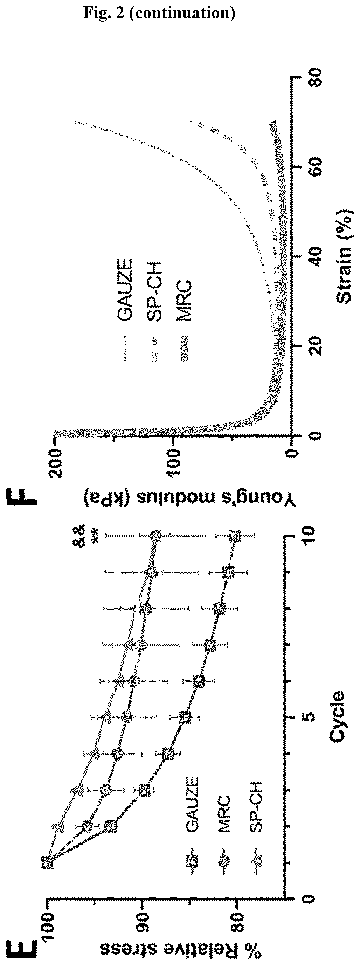

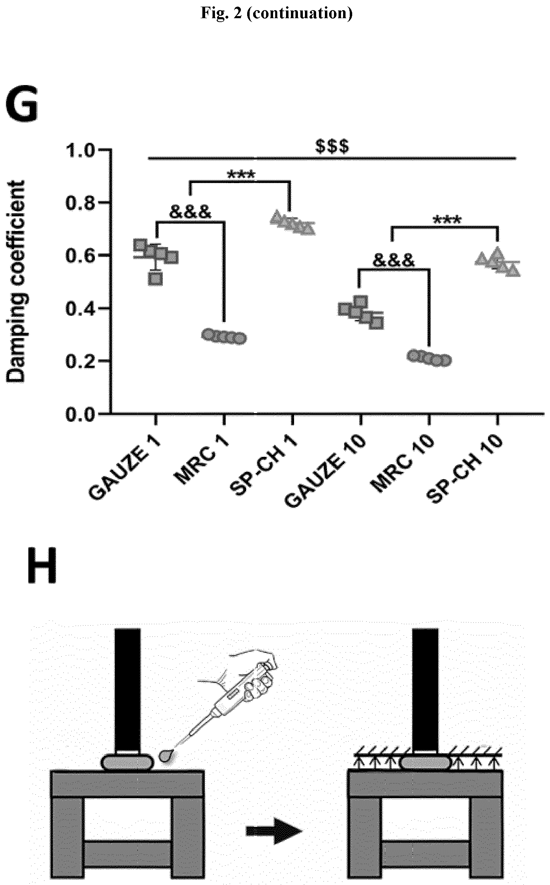

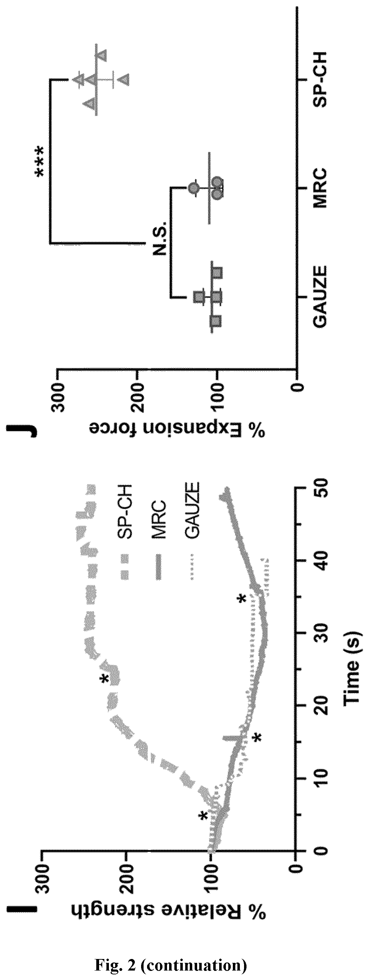

**Figure 2.** Mechanical characterization. (A) Scheme of the procedure carried out for the cyclic compression test. (B-D) Cyclic compression stress-strain curves of gauze (B), MRC (C) and SP-CH (D). (E) Relative stress (%) of the materials for 10 cyclic compressions. Error bars, mean $\pm$ SD. && indicates p < 0.01 by comparing between the gauze and MRC groups. ** indicates p < 0.01 by comparing between the gauze and SP-CH groups. (F) Young's modulus of the materials at different strains. (G) Damping coefficients of the material for cycles 1 and 10. Error bars, mean $\pm$ SD. &&& indicates p < 0.001 by comparing between the gauze and MRC groups. *** indicates p <0.001 by comparing SP-CH to the other groups. $$$ indicates p <0.001 by comparing between cycle 1 and cycle 10 of the same group. (H) Scheme of the procedure carried out for the expansion force test. (I) Relative expansion strength curves of the materials. * indicates the moment at which water was added to each material. (J) Relative expansion force of the materials. Error bars, mean $\pm$ SD. *** indicates p <0.001 by comparing SP-CH to the other groups. N.S., nonsignificant (p > 0.05).

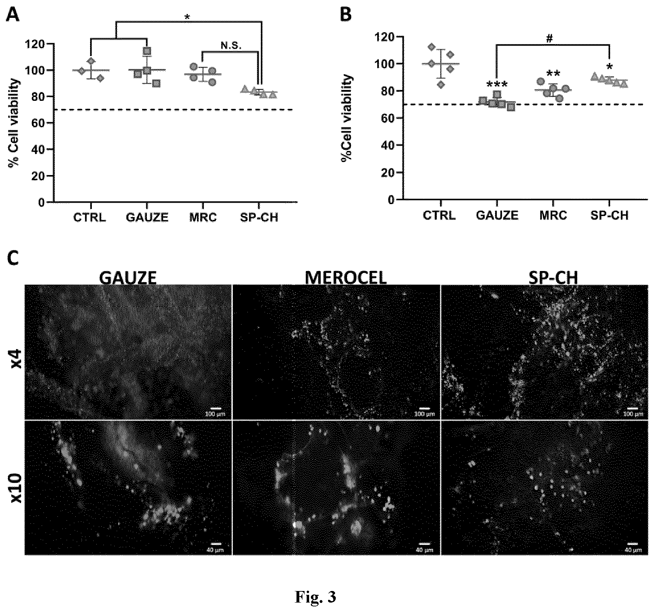

**Figure 3.** Cytotoxicity studies. (A) Direct cytotoxicity. (B) Indirect cytotoxicity. The line marks the 70% cell viability. Error bars, mean $\pm$ SD. N.S., nonsignificant (p > 0.05). * indicates p < 0.05; ** indicates p < 0.01; *** indicates p <0.001 in comparison with the control group. # indicates p < 0.05; by comparing between the groups. (C) Live/Dead micrographs of the cell-cultured materials, with calcein-etidium staining. Scale bars are 100 and 40 $\mu$m in the above and below rows, respectively.

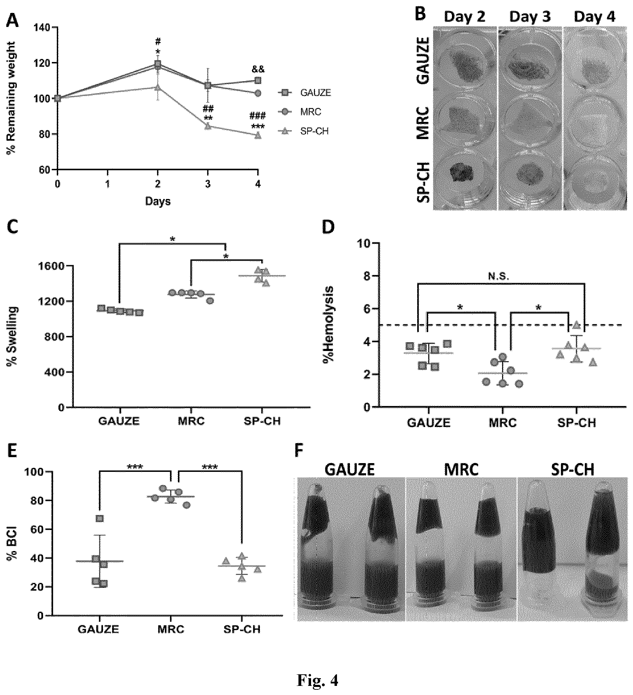

**Figure 4.** Blood performance. (A) Degradation in blood. Error bars, mean $\pm$ SD. && indicates p < 0.01 by comparing between the gauze and MRC groups. # indicates p < 0.05; ## indicates p < 0.01; ### indicates p < 0.001 by comparing between MRC and SP-CH groups. * indicates p < 0.05; ** indicates p < 0.01; *** indicates p < 0.001 by comparing between the gauze and SP-CH groups. (B) Images of the plates corresponding to blood degradation after 2, 3, 4 and 7 days. (C) Maximum blood swelling capacity. Error bars, mean $\pm$ SD. * indicates p < 0.05 by comparing between the groups. (D) Hemolysis assay. The line marks 5% hemolysis. Error bars, mean $\pm$ SD. N.S., nonsignificant (p > 0.05). * indicates p < 0.05 by comparing between the groups. (E) Blood Clotting Index of the materials. Error bars, mean $\pm$ SD. *** p indicates <0.001 by comparing between the groups. (F) Image of the tube inversion method for visually assessing blood coagulation.

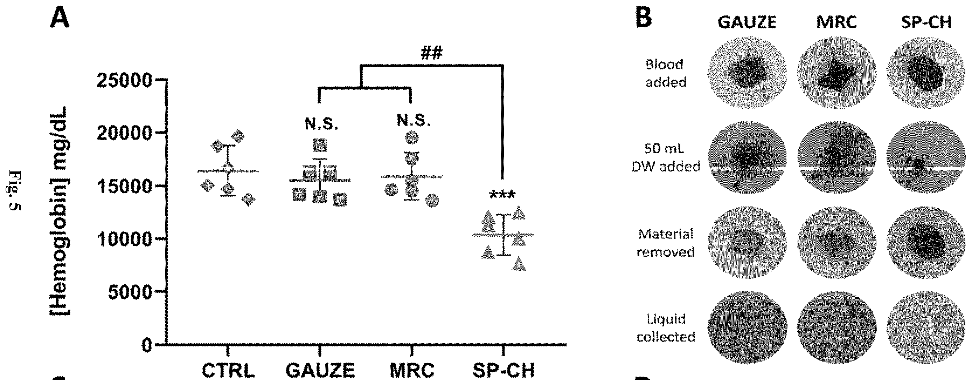

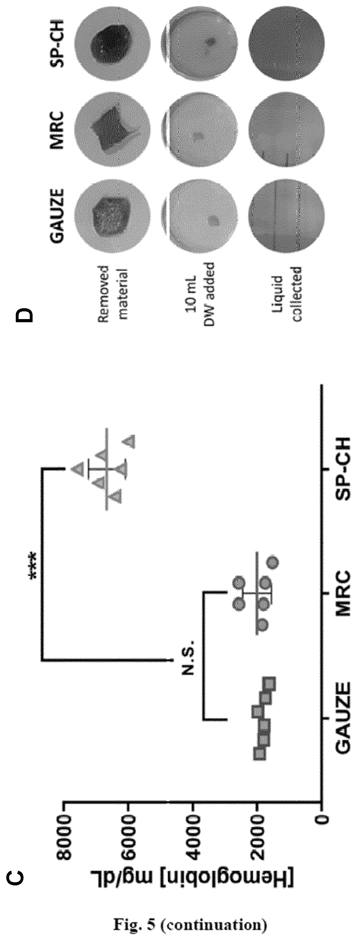

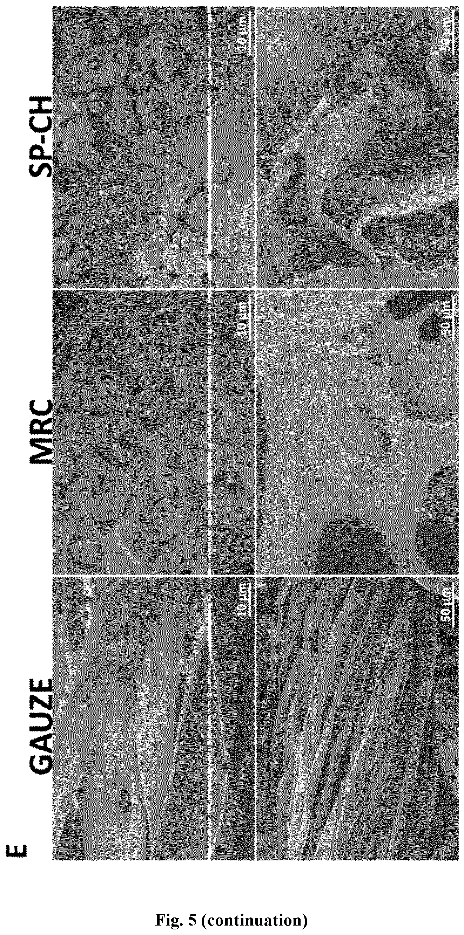

**Figure 5.** RBC adhesion. (A) Hemoglobin outside the materials. Error bars, mean $\pm$ SD. N.S., nonsignificant (p > 0.05). *** p indicates <0.001 by comparing with the control group; ## indicates p < 0.01 by comparing between the groups. (B) Photographs of the appearance of the materials at different stages of the determination of hemoglobin outside the materials. (C) Hemoglobin within the materials. Error bars, mean $\pm$ SD. N.S., nonsignificant (p > 0.05). *** p indicates <0.001 by comparing between groups. (D) Photographs of the appearance of the materials at different stages of the determination of hemoglobin within the materials. (E) SEM micrographs of RBCs adhered to the surface of the materials.

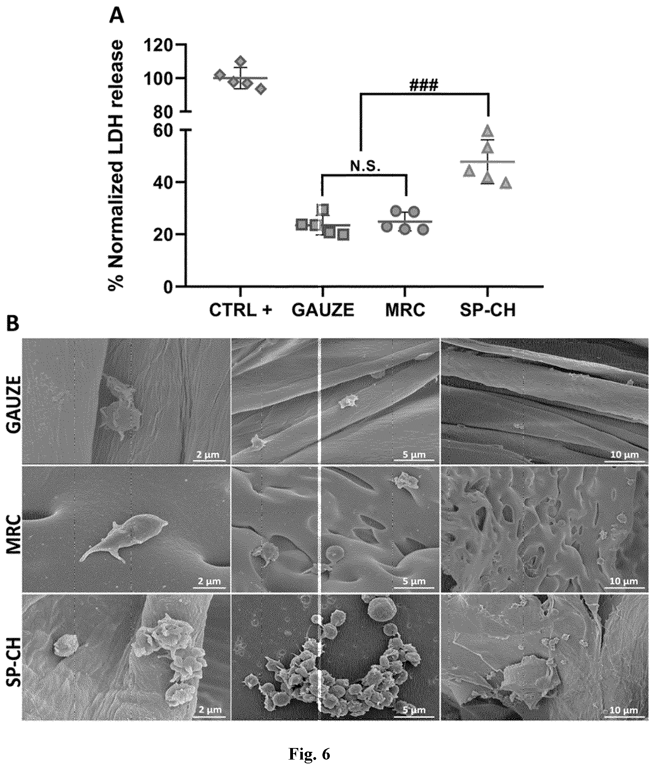

**Figure 6.** Platelet adhesion. (A) Normalized LDH release (%) of the materials. Error bars, mean $\pm$ SD. N.S., nonsignificant (p > 0.05). ### indicates p < 0.001 by comparing between the groups. n=5 independent samples per group. (B) SEM micrographs of platelets adhered to the surface of the materials.

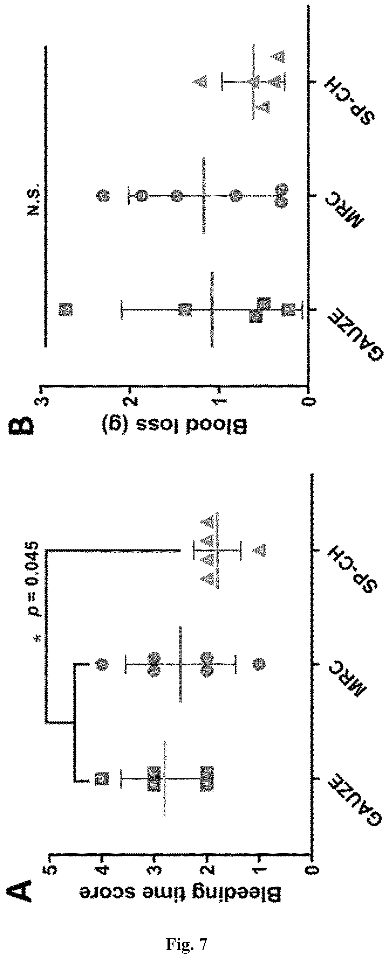

**Figure 7.** In vivo hemostatic efficacy. (A) Bleeding time score of the three materials. Error bars, mean $\pm$ SD. * indicates p < 0.05 by comparing SP-CH with the other materials. (B) Blood loss (g). Error bars, mean $\pm$ SD. N.S., nonsignificant (p > 0.05).

## DETAILED DESCRIPTION OF THE INVENTION

The scope of protection is defined by the claims.The references to methods of treatment are to be interpreted as references to the compounds, pharmaceutical compositions and medicaments of the present invention for use in a method for treatment of the human (or animal) body by therapy.

The present description relates to the invention of a sponge-like scaffold (SLS) comprising soy protein isolate (SPI), chitin (CH) and a plasticizer for use as a promoter of haemostasis. The inventors of the present invention have determined that the structural characteristics of the SLS, together with their physio and chemical properties make the SLS an ideal material for promoting haemostasis processes and as such for use in products designed to be implemented in the treatment of bleeding events.

### *Use of the sponge-like scaffold*

A first aspect of the present invention relates to a SLS comprising SPI, CH and a plasticizer, from here onwards the SLS of the invention, for use in promoting haemostasis.

The term "sponge-like scaffolds", also referred to by its acronym SLS, as used herein refers to a structure composed of one or more components which mimics the extracellular matrix (ECM) of an animal body which provides structural support and biochemical support to the surrounding cells. Said SLS can be characterized by structural properties such as porosity, pore size, degradation, swelling profile, deformation resistance, damping coefficient and other mechanical properties, as well as physicochemical properties such as swelling capacity and degradability, and by biomedical properties such as cytotoxicity, and biocompatibility amongst others.

In a preferred embodiment of the use of the invention the SLS of the invention has a porosity of around between 65% and 95%, preferably of around between 70% and 80%, more preferably the average porosity of the SLS of the invention is of around 65%, 66%, 67%, 68%, 69%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94% or 95%. The term "porosity" as used herein refers to the void fraction of a material, i.e., the empty space in a material and is a fraction (or a percentage) of the volume of voids over the total volume. Methods to measure porosity are known to the skilled person in the field. Examples of such methods are, without limitation, mercury porosimetry, gas adsorption (such as nitrogen adsorption) and liquid displacement using ethanol 98% as exemplified below in Example 1.1.

Another mechanical characteristic important for the function of the SLS is the pore size of said scaffold. The "pore size" as used in the present context relates to the size of each individual void in the SLS. The size of individual pores may determine the hemostatic properties of the SLS and a balance between large pore size, which allow red blood cell and platelet internalization, and small pores size that provide optimal surface area, improving cell adhesion, should be sought. As such, in a preferred embodiment of the use of the invention, the SLS has a pore size of around between 0.5 millimeters (mm) and 1.5 mm, preferably an average pore size of around 0.6 mm, 0.7 mm, 0.8 mm, 0.9 mm, 1 mm, 01.1 mm, 1.2 mm, 1.3 mm, 1.4 mm, 1.5 mm. Similarly to porosity, methods to measure pore size are also well known to the skilled person in the field and include, without limitation, analysis of images from transmission electron microscopy or micrographs from scanning electron microscopy as exemplified below in Example 1.1.

The SLS of the invention comprises three components, namely SPI, CH and a plasticizer.

The term "soy protein isolation", or its acronym "SPI", as used herein refer to the compound obtained from soy flour, a by-product of the manufacturing process of the soybean industry. SPI, generally is obtained from defatted soy flour with a solubility in water by extraction in a solution with a pH below 9. Once the extract is recovered, it is clarified to remove the insoluble material and the supernatant liquid is acidified to a pH range of 4-5. The precipitated protein-curd is collected and separated from the whey by centrifugation. In a preferred embodiment of the scaffold for use according to the invention the SPI compound comprises at least 85%, 86%, 87%, 88%, 89%, 90%, 91%, 92%, 93%, 94%, 95%, 96%, 97%, 98%, 99% or 100% of protein, preferably 90% of protein. SPI is an abundant biomaterial that shows biocompatibility and low immunogenicity, in part, due to its amino acid and chemical composition. Therefore, in another embodiment of the use of the invention, the SPI compound comprises glutamic acid (Glu), aspartic acid (Asp), leucine (Leu), glycine (Gly), alanine (Ala) and proline (Pro), in a percentage with respect to the total amino acid residues present in the SPI, of at least 6%, 7%, 8%, 9%, 10% or 11% each, preferably at least 6% each. The person skilled in the art will know how to determine the amino acid content of SPI as well as the elemental composition. Examples of techniques which can be used to determine the amino acid content in a given material are ion exchange chromatography coupled with detection of amino acids in a post column derivatization with ninhydrin reagent (Amino Acid Analyzer) and attenuated total reflection Fourier infrared spectroscopy (ATR-FTIR) and X-ray photoelectron spectroscopy (XPS).

The SPI also possesses sulphur which has an important function in forming inter-chain and intra-chain disulphide bonds and therefore is important for the structural stability of the SLS. In a prefer embodiment of the use of the invention, the SPI compound has a sulphur content of around less than 2%, preferably of around less than 1.5%, 1.4%, 1.3%, 1.2%, 1.1%, 1.0%, 0.9%, 0.8%, preferably around 1%.

To determine the elemental composition of SPI, spontaneous combustion with subsequent chromatographic separation and is a non-limiting example of a technique that can be used.

In one embodiment, the SPI for use according to the invention comprises Glu, Asp, Leu, Ser, Gly, Ala and Pro, in a percentage with respect to the total amino acid residues present in the SPI, of at least 6% each.

SPI contains elements that promote haemostasis by way of its chemical interaction with cellular and humoral components of the coagulation pathway. In particular, SPI is a source of phylloquinone or vitamin K1 which serves as a cofactor for the endoplasmic enzyme γ-glutamate carboxylase (GGCX), which is essential for the carboxylation of certain protein-bound glutamate residues into γ-carboxyglutamate (Gla). Additionally, the SPI present in the sponge has RGD-motifs which allows its binding to platelets.

It is accordingly evident that properties of the SLS of the invention depend on its chemical/molecular characteristics of its components, in addition to its sponge-like structure, which affects its hemostatic properties.

The term "chitin" or the acronym "CH" as used herein refers to a long-chain polymer of β-(1-4)-N-acetyl-D-glucosamine, with a structure $(C_8H_{13}O_5NH_2O)_n$ with 2-acetamido-2-deoxy-β-D-glucose (NAG) monomer units, which are attached to each other via β(1→4) linkages.

CH occurs as crystalline microfibrils in nature that form structural elements of the exoskeletons of arthropods, fungal cell walls amongst others. Therefore, in a preferred embodiment of the use of the invention the CH is obtained from squid pen. The term "squid pen" as used herein refers to the gladius, also known as "pen", a hard internal body part found in many cephalopods of the superorder Decapodiformes, particularly squid, which is composed of mostly chitin.

CH has three different crystalline forms: $\alpha$-, $\beta$- and $\gamma$-forms. These differ in the orientation of the micro-fibrils. The commonest form of chitin is $\alpha$-chitin, whose micro-fibrils are orientated in an antiparallel way. $\beta$-chitin is a less common form of chitin whose micro-fibrils are orientated in a parallel way. Finally, $\gamma$-chitin possess micro-fibrils in both parallel and anti-parallel orientations. In a preferred embodiment of the use of the invention the CH is selected from a group consisting of: $\alpha$-chitin, $\beta$-chitin, $\gamma$-chitin or any combination thereof. In a more preferred embodiment the CH contains $\beta$-chitin.

As to obtain the SLS of the invention the content of CH is determined in relation to the content of SPI. Therefore, in a preferred embodiment of the use of the invention, the SLS of the invention has a content of CH of about between 20% to 40% weight to weight (w/w) with respect to the dry SPI content, preferably about between 25% to 35% w/w with respect to the dry SPI content, more preferably about 30% w/w with respect to the dry SPI.

Natural CH has different levels of deacetylation, giving a continuum of structure between chitin (fully acetylated) and chitosan (fully deacetylated). Although either acids or alkalis can be used to deacetylate CH, the fact that glycosidic bonds are more susceptible to acid which would destroy the chain, the alkali deacetylation process is used more frequently. In a preferred embodiment of the use of the invention the CH is obtained by an alkaline hydrolysis at room temperature.

Natural CH, in general, has a degree of acetylation of around 90%, while chitosan is a fully or partially N-deacetylated derivative with a typical degree of deacetylation of more than 65%. Fully deacetylated chitin becomes soluble in dilute acetic and formic acids. In a preferred embodiment of the use of the invention, the CH has an acetylation degree of at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%, preferably 97%. In another preferred embodiment of the use of the invention, the SLS is substantially devoid of chitosan. The expression "substantially devoid of chitosan as used herein refers to the fact that the CH used in the SLS of the invention has a minimal content of chitosan of less than 10%, preferably less than 10%, 9%, 8%, 7%, 6%, 5%, 4%, 3%, 2%, 1.5%, 1%, 0.9%, 0.8%, 0.7%, 0.6%, 0.5%, 0.4%, 0.3%, 0.2%, 0.1%.

The level of acetylation of CH can be determined by several techniques which the person skilled in the art will be aware of, such as, and without limitation, IR spectroscopy, pyrolysis gas chromatography, gel permeation chromatography, UV-vis spectrophotometry, H NMR spectroscopy, C solid state NMR, thermal analysis and X-ray analysis.

The term "plasticizer" as used herein refers to a chemical that increases the rheological property of the SLS, such as viscosity and elasticity/flexibility. The content of the plasticizer in the SLS is determined in relation to the content of SPI. Therefore, in a preferred embodiment of the use of the invention, the SLS of the invention has a content of plasticizer of about between 10% to 50% weight to weight (w/w) with respect to the dry SPI content, preferably about 20% w/w with respect to the dry SPI content, more preferably about 30% w/w with respect to the dry SPI.

In a preferred embodiment the plasticizer is a polyol. The term "polyol" in the present context is synonymous with "sugar alcohol", "polyhydric alcohol", and "polyalcohol" and refers to a hydrogenated form of carbohydrate, whose carbonyl group (aldehyde or ketone, reducing sugar) has been reduced to a primary or secondary hydroxyl group (hence the alcohol), such as, e.g., mannitol from mannose, xylitol from xylose, and lactitol from lactulose. Polyols have the general formula $H(HCHO)n +1H$. Both monosaccharides and disaccharides can form polyols; however, polyols derived from disaccharides are not entirely hydrogenated because only one aldehyde group is available for reduction. Non-limiting examples of polyols include glycerol, erythritol, threitol, arabitol, erythritol, ribitol, xylitol, galactitol (or dulcitol), gluctiol (or sorbitol), iditol, inositol, mannitol, isomalt, lactitol, maltitol, and polyglycitol. Therefore, in a preferred embodiment of the use of the invention the plasticizer is a polyol selected from a group consisting of: glycerol, erythritol, threitol, arabitol, erythritol, ribitol, xylitol, galactitol, gluctiol, iditol, inositol, mannitol, isomalt, lactitol, maltitol, polyglycitol or any combination thereof. In another embodiment of the use of the invention the plasticizer is glycerol.

The surface area of the SLS relates to the capacity a material has to absorb fluids such as water or blood present in hemorrhages, i.e., its "swelling capacity". In another preferred embodiment of the use of the invention, the SLS has a swelling maximum capacity of around between 1000% to 1800% of its initial weight, preferably of around 1050%, 1100%; 1150%, 1200%, 1250%, 1300%, 1350%, 1400%, 1450%, 1500%, 1550%, 1600%, 1650%, 1700%, 1750% or 1800% of its initial weight, more preferably of around 1,442% of its initial weight. In another preferred embodiment the SLS has swelling capacity profile of around between 1000% to 1800% of its initial weight within 5 seconds, preferably of around 1050%, 1100%; 1150%, 1200%, 1250%, 1300%, 1350%, 1400%, 1450%, 1500%, 1550%, 1600%, 1650%, 1700%, 1750% or 1800% of its initial weight within 5 seconds, more preferably 1,383 of its initial weight within 5 seconds. The swelling capacity can be determined as exemplified below in the Examples section.

The terms "degradability" or "hydrolytic degradation" as used herein refers to the property of the material which is reabsorb by or lost to the tissue around it while performing its function. It is a relevant property for the moment of extracting the material from the subject, such as a nasal pack which is removed from the nostril, as it reduces both patient discomfort and rebleeding upon removal, among other advantages. Degradability or hydrolytic degradation can be determined as explained in Example 2.3 of the present description. In a preferred embodiment of the use of the invention, the SLS has a degradability of around 15 to 30% of its initial weight after 48h, preferably of around 15%, 16%, 17%, 18%, 19%, 20%, 21%, 22%, 23%, 24%, 25%, 26%, 27%, 28%, 29% or 30% of its initial weight after 48h.

The term "deformation resistance" in the context of the present invention refers to the capacity of the SLS to maintain its strength and viscoelasticity without losing resistance in repeated cycles of compression, which can be measured by a cyclic compression test wherein the material is subjected to a degradation force for 10 cycles and the loss of resistance to deformation can be measured as a percentage of the maximum stress required to deform the material in cycle 1 in comparison with cycle 10 - relative maximum stress. In a preferred embodiment of the use of the invention, the SLS has a relative stress of around between 80% and 95% after 10 cycles, preferably the average porosity of the SLS of the invention is of around 80%, 81%, 82%, 83%, 84%, 85%, 86%, 87%, 88%, 89%, 90%, 91%, 92%, 93%, 94% or 95% after 10 cycles.

The SLS can be subject to mechanical compression and as such its capacity to dissipate vibrational energy, and therefor protect from impact compression waves, is relevant for the function of the material. This property is measured by the "damping coefficient", which again is measured during cycles of deformation of the material as a ration of energy dissipation. In a preferred embodiment of the use of the invention, the SLS has a damping coefficient of around 0.65 and 0.80 in the first cycle of compression, preferably 0.65, 0.66, 0.67, 0.68, 0.69, 0.7, 0.71, 0.72, 0.73, 0.74, 0.75, 0.76, 0.77, 0.78, 0.79 or 0.80 in the first cycle of compression. In another preferred embodiment of the use of the invention, the SLS has a damping coefficient of around 0.50 and 0.60 in the tenth cycle of compression, preferably 0.5, 0.51, 0.52, 0.53, 0.54, 0.55, 0.56, 0.57, 0.58, 0.59 or 0.6 in the tenth cycle of compression, more preferably 0.57 in the tenth cycle of compression.

Another mechanical property of SLS is the capacity of expanding outwards when absorbing liquid, referred to as "expansion force" in the present description. The expansion force is determined by an expansion force test (see Example 1.4) and is presented as a percentage of the initial strength of the material. In a preferred embodiment of the use of the invention, the SLS has an expansion force of around between 225% to 275% in respect to the initial material strength, preferably of around 225%, 230%; 235%, 240%, 245%, 250%, 250%, 255%, 265%, 270% or 275% of its initial weight, more preferably of around 250% of its initial weight.

Methods to determine the mechanical properties of the SLS of the invention, namely pore size, porosity, swelling capacity, degradability, deformation resistance, damping coefficient and expansion force, are exemplified in the Examples section of the present description.

The SLS of the invention in addition has good hemocompatibility properties as indicated by its hemolysis degree. The term "hemolysis" as used herein refers to the destruction of red blood cells (RBCs) due to shear stress or changes in osmotic pressure. In a preferred embodiment of the use of the invention the SLS has a hemolysis rate of less than 5%, preferably less than 4%.

The SLS of the invention is used to promote haemostasis and therefore it promotes blood clothing. Therefore, in a preferred embodiment of the use of the invention the SLS has a blood clotting index (BCI) of around between 15% and 55%, preferably of around 15%, 16%, 17%, 18%, 19%, 20%, 21%, 22%, 23%, 24%, 25%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54% or 55%, more preferably 37%.

*Method for obtaining the SLS* (not according to the claimed invention)

As mentioned previously the SLS of the invention is composed by the SPI, CH and plasticizer compounds. Accordingly, in a preferred embodiment of the use of the invention the SLS has been obtained by a method, from here onwards the method of the invention, that comprises:

> (i) Forming a mixture comprising SPI, CH and a plasticizer at a basic pH,
> (ii) Forming a 3D structure by pouring the mixture obtained in step (ii) into moulds or 3D printing and
> (iii) Freeze drying the material obtained in step (ii).

The expression "Forming a mixture comprising SPI, CH and a plasticizer at a basic pH" of step (i) of the method of the invention as used herein refers to the process of bringing together the three compounds that form the SLS, i.e., SPI, CH and plasticizer, into a mix or blend such as a solution where the solvent may be the plasticizer itself or any other adequate solvent, such as distilled water, water type I or type II. In a preferred embodiment of the use of the invention, step (i) of the method of the invention further comprises a solvent, preferably distilled water.

Once mixed together, the pH of the mixture is adjusted to be greater than 6.5. In a preferred embodiment of the use of the invention, in step (i) of the method of the invention the pH is greater than 6.5, preferably at least 8, 9, 10, 11, 12, 13 or 14, more preferably the pH is 10.

In order to obtain a homogeneous mixture, said mixture may be incubated and stirred. In a preferred embodiment of the use of the invention, in step (i) of the method of the invention the mixture is heated to around 60 degrees (°C) to 90°C for 15 minutes (min) to 45 min, preferably 80 °C for 30 min, under stirring, preferably magnetic stirring. In a more preferred embodiment of the use of the invention in step (i) of the method of the invention, the heating and stirring process may be repeated once or twice.

The expression "forming a 3D structure by pouring the mixture obtained in step (ii) into moulds or 3D printing" as used herein refers to the process of obtained a tridimensional object using the mixture obtained in step (i) of the method of the invention, whereby that process involves shaping the mixture by placing it in a hollow container, i.e., a mould, which has the structure desired and let it solidify or 3D printing into the desired structure.

The term "3D printing" as used herein refers to additive manufacturing methods that employ a computer-controlled printer head and/or translation stage, which moves a pattern-generating device in the form of one or more deposition nozzle(s) to fabricate materials according to a pre-determine (pre-programmed) form.

Once obtained the desired mixture and having it poured into molds or 3D printed to obtain the desired 3D shape, the next step of the process is to freeze dry the material obtained. The term "freeze drying", also known as lyophilisation or cryodesiccation, as used herein refers to the process of dehydration at low temperature, which involves freezing the product, lowering the pressure and then removing the ice by sublimation. In a preferred embodiment of use of the invention, step (iii) of the method of the invention is performed by firstly placing the material obtained in step (ii) at a temperature of around between -30 °C to -10 °C for 24 hours to 72 hours, by preferably -22 °C for 48 hours, followed by leaving the material at a temperature of around between -30 to -10 °C for 48h to 96 h, preferably -22°C for 72h.

In a preferred embodiment of the use of the invention, the SPI used in step (i) of the method of the invention comprises at least 85%, 86%, 87%, 88%, 89%, 90%, 91%, 92%, 93%, 94%, 95%, 96%, 97%, 98%, 99% or 100% of protein, preferably 90% of protein.

In a preferred embodiment of the use of the invention, the SPI used in step (i) of the method of the invention comprises Glu, Asp, Leu, Gly, Ala and Pro, in a percentage with respect to the total amino acid residues present in the SPI, of at least 6%, 7%, 8%, 9%, 10% or 11% each. In another preferred embodiment of the use of the invention, the SPI used in step (i) of the method for obtaining the SLS has a sulphur content of around less than 2%, preferably of around less than 1.5%, 1.4%, 1.3%, 1.2%, 1.1%, 1.0%, preferably less than 1%.

In another preferred embodiment of the use of the invention, the the CH used in step (i) of the method of the invention is selected from a group consisting of: $\alpha$-chitin, $\beta$-chitin, $\gamma$-chitin or any combination thereof, preferably $\beta$-chitin.

In another preferred embodiment of the use of the invention, the CH used in step (i) of the method of the invention has an acetylation degree of at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%, preferably 97%.

In another preferred embodiment of the use of the invention, the CH used in step (i) of the method of the invention is that obtained from a squid pen.

In another preferred embodiment of the use of the invention, the the CH used in step (i) of the method of the invention is obtained from a squid pen by a method that comprises:

(i) treating the squid pen under alkaline conditions, and
(ii) washing the solid fraction.

The expression "Treating the squid pen with an alkaline solution" in step (i) of the method for obtaining the CH from a squid pen, as used herein refers to the process of obtaining CH from the squid pen by an alkaline hydrolysis. Said alkaline hydrolysis leads to the deacetylation of CH. The term "alkaline solution" as used herein refers to a solution which has an alkali, i.e., a basic, ionic salt of an alkali metal dissolved in water and whose pH of the solution is greater than pH 7. Examples of said ionic salts, without limitation, are sodium hydroxide (NaOH), potassium hydroxide (KOH), calcium hydroxide ($Ca(HO)_2$) and magnesium hydroxide ($Mg(HO)_2$). In a preferred embodiment of the use of the invention, the alkaline solution of step (i) of the method to obtain CH from a squid pen comprises an ionic salt selected from: sodium hydroxide (NaOH), potassium hydroxide (KOH), calcium hydroxide ($Ca(HO)_2$), magnesium hydroxide ($Mg(HO)_2$) and any combination thereof, preferably NaOH. The concentration necessary of said ionic salt in a solution is the sufficient to attain the desired effect of obtaining CH. In a preferred embodiment of the use of the invention, the alkaline solution of step (i) of the method to obtain CH from a squid pen comprises an ionic salt at 1 Molar (M), preferably NaOH at 1 M.

Likewise, in order to minimize the deacetylation of the native CH, the volume and incubation conditions required of the alkaline solution is also the sufficient to obtain CH from the squid pen, and the person skilled in the field will be able to easily determine such volume using common experimental techniques. In a preferred embodiment of the use of the invention, the alkaline solution of step (i) of the method to obtain CH from a squid pen is used M in a relation of 1/15 to 1/25 weight to volume (w/v) to the weight of the squid pen, preferably 1/16 w/v, 1/17 w/v, 1/18 w/v, 1/19 w/v, 1/20 w/v, 1/21 w/v, 1/22 w/v, 1/23 w/v or 1/24 w/v, more preferably 1/20 w/v. In another preferred embodiment of the use of the invention, step (i) of the method to obtain CH from a squid pen is carried out at room temperature for around 12h to 36h, preferably 24h, under continuous stirring. The term "room temperature" as used herein and part of the general knowledge for a person skilled in the art, refers to a temperature in the range of 20 °C to 25 °C.

In another preferred embodiment of the use of the invention, step (ii) of the method to obtain CH from a squid pen is carried out with distilled water until neutral pH. The term "neutral pH" as used herein refers to a pH which is around between 6.5 and 7.5, preferably the pH is 7 pH.

In the context of the use of the invention the SLS of the invention can be used in a variety of forms and dispositions. In a preferred embodiment of the use of the invention, the SLS is provided in a form selected from a group consisting of: dry, lyophilized, fully hydrated, partially hydrated or impregnated, preferably lyophilized.

The term "dry" as used herein refers to the removal of all of the content of water present in the SLS by methods which are well known for the person skilled in the art, such as, and without limitation, hot hair drying, contact drying, spray drying and vacuum drying.

The term "lyophilized" as used herein refers to the process of freeze drying which has been previously described.

The terms "fully hydrated" and "partially hydrated" as used herein refers to SLS whose content of water is maximum, i.e., full wherein no more water can be uptake by the material, or the content is above zero and below the maximum content or water, i.e., partial.

The term "impregnated" as used herein refers to the soaking or saturation of the SLS with a solution which contains an agent of interest. Said agent can be simple saline solution or a therapeutically agents which, for example and without limitation, promotes haemostasis, inflammation reduction, is a biocide, virucide or fungicide, etc. Therefore, in a preferred embodiment of the use of the invention, the SLS is provided in a form impregnated with a solution with an agent of interest, wherein said agent is selected from a group consisting of: analgesics, steroids, antihistamines, anesthetics, bactericides, disinfectants, fungicides, vasoconstrictors, hemostatics, chemotherapeutic drugs, antibiotics, keratolytics, cauterizing agents, antiviral drugs, epidermal growth factor, fibroblast growth factors, transforming growth factors, glycoproteins, collagen, fibrinogen, fibrin, humectants, preservatives, lymphokines, cytokines, odor controlling materials, vitamins, clotting factors or any combination thereof.

*Therapeutic uses in haemostasis*

The SLS of the invention finds use in promoting haemostasis, the process of stopping bleeding. Therefore, in an embodiment of the use of the invention the promoting of haemostasis is in the treatment of a bleeding wound.

The term "treatment" as used herein refers to an intervention (e.g. the administration of the SLS of the invention) which cures, ameliorates or lessens the symptoms of a disease or removes (or lessens the impact of) its cause(s) (for example, the reduction or stoppage of nasal haemorrhage). In this case, the term is used synonymously with the term "therapy". In the context of the present invention the use of the invention in the treatment of a bleeding wound the SLS is used in a therapeutically effective amount.

The expression "therapeutically effective amount" as used herein defines an amount that can be administered or applied to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility, reduced or stoppage of bleeding and other markers of clinical improvement. A therapeutic result need not be a complete cure.

The term "bleeding" as used herein refers to the loss of blood from a damaged blood vessel. The term "wound" as used in the present description refers to any open wound which presents with blood flow or loss, regardless of the stage or depth of the wound.

Examples of wounds that can be treated according to the present invention are, for example, open wounds which present blood loss or flow. Open wounds which can be treated by the use of the invention include, without limitation, burns caused cold or heat, incisions, ulcers, sores, lacerations, abrasions, acne, bite wounds, punctures or ballistic wounds.

Aseptic wounds, contused wounds, incised wounds, lacerated wounds, open wounds, penetrating wounds, perforating wounds, punctured wounds, septic wounds, subcutaneous wounds, etc. can also be treated according to the present invention. Examples of sores are decubitus ulcers, aphthae, chrome ulcers, cold ulcers, pressure ulcers, etc. Examples of ulcers are, for example, peptic ulcer, duodenal ulcer, gastric ulcer, gouty ulcer, diabetic ulcer, hypertensive ischemic ulcer, stasis ulcer, ulcus cruris (venous ulcer), sublingual ulcer, submucosal ulcer, symptomatic ulcer, trophic ulcer, tropical ulcer and venereal ulcer, for example caused by gonorrhea (including urethritis, endocervicitis and proctitis). The conditions related to wounds or sores which can be successfully treated according to the invention are burns, anthrax, tetanus, gaseous gangrene, scarlatina, erysipelas, sycosis barbae, folliculitis, impetigo contagiosa or bullous impetigo, etc. There is frequently an overlapping between the use of the terms "wound" and "ulcer" and "wound" and "sore" and, moreover, the terms are often used randomly. Therefore, as has been previously mentioned, in the present context the term "wound" includes the terms "ulcer", "lesion'", "sore" and "infarction" and the terms are used indistinctly unless otherwise indicated.

The types of wounds to be treated according to the invention also include i) general wounds, such as, for example, surgical, traumatic, infectious, ischemic, thermal, chemical and bullous wounds; ii) specific wounds of the oral cavity, such as, for example, wounds after extractions, endodontic wounds especially in relation to the treatment of cysts and abscesses, ulcers and bacterial, viral or autoimmune lesions, mechanical, chemical, thermal, infectious and lichenoid wounds; herpetic ulcers, aphthous stomatitis, acute necrotizing ulcerative gingivitis and burning mouth syndrome are specific examples; and iii) wounds on the skin, such as, for example, neoplasias, burns (for example, chemical or thermal burns), lesions (bacterial, viral, autoimmune), bite wounds and surgical incisions. Another way to classify the wounds is as i) small loss of tissue due to surgical incisions, minor abrasions and minor bite wounds, or as ii) significant loss of tissue. This latter group includes ischemic ulcers, pressure sores, fistulas, lacerations, severe bite wounds, thermal burns and wounds in donor site (in soft and hard tissues) and infarctions.

In a preferred embodiment of the use of the invention the promotion of haemostasis is in the treatment of a bleeding wound wherein the wound is selected from the group consisting of: aseptic wound, contused wound, incised wound, lacerated wound, open wound, penetrating wound, perforating wound, punctured wound, septic wound, subcutaneous wound, fistula wound and bite wound.

In another preferred embodiment of the use of the invention the promotion of haemostasis is in the treatment of a bleeding wound, wherein the bleeding wound is a haemorrhage. The term "haemorrhage" as used in the herein refers to a moderate to heavy release of blood within or from a body. The term haemorrhage in the context of the present invention should be interpreted broadly to include arterial, venous and capillary haemorrhage, as well as, to include primary and secondary haemorrhage and internal and external haemorrhage. Haemorrhage can be classified into four classes depending on the amount of blood loss of the total volume of blood of the body. Class I haemorrhage involves up to 10 - 15% of blood volume loss; Class II involves 15 - 30 % blood volume loss; Class III haemorrhage involves 30 - 40% of blood volume loss; and Class IV involves more than 40% blood volume loss. Haemorrhage can be cause by any of the types of wounds previously describe. There is frequently an overlapping between the use of the terms "bleeding" and "haemorrhage" and, moreover, the terms are often used randomly. Therefore, as has been previously mentioned, in the present context the term "bleeding" includes the terms "haemorrhage" and the terms are used indistinctly unless otherwise indicated. Examples of haemorrhage that can be treated in the context of the invention are, without limitation, epistaxis or nasal haemorrhage, tooth eruption, postoperative haemorrhage, oral haemorrhage, gingival haemorrhage or surgical haemorrhage. In a preferred embodiment of the use of the invention the promotion of haemostasis is in the treatment of haemorrhage wherein the haemorrhage is selected from a group consisting of: nasal haemorrhage, tooth eruption, postoperative haemorrhage, oral haemorrhage, gingival haemorrhage or surgical haemorrhage, preferably nasal haemorrhage.

In the present invention context any animal which presents with blood loss can be treated by the SLS of the invention. Therefore, in another embodiment of the use of the invention, the bleeding wound is from a subject, preferably an animal, more preferably a mammal. Mammals include, without limitation, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In a preferred embodiment of the use of the invention the subject is a human or any age, gender or race.

*Products*

The SLS previously defined in the present description finds application in the elaboration of products which can be used for promoting haemostasis in a subject. Therefore another aspect of the present invention relates to a haemostatic product, from here onwards the product of the invention that comprises a SLS of the invention and a support wherein the SLS is immobilized in a support.

The skilled person in the field will know the indicated support that can be used with the SLS of the invention and the best procedure to apply the SLS to said support. Examples of supports are, without limitation, gauze, tissue, plastic tissue, stick, string, film, plaster, surgical dressing or bandage. In a preferred embodiment of the product of the invention the support is selected from a group consisting of: gauze, tissue, plastic tissue, stick, string, film, plaster, surgical dressing or bandage.

The term "gauze" as used herein refers to a very thin, light, loosely woven material, usually of silk or cotton, and also is meant to include other material of similar open texture such as wire gauze. The term "gauze" as used herein also includes zinc-gauzes (gauzes with zinc impregnated).

The term "tissue" as used herein refers to bioactive wound dressings which are produced from biomaterials of natural or artificial sources. Examples of such tissues are, without limitation, collagen, hyaluronic acid, chitosan, alginate and elastin.

The term "plastic tissue" as used herein refers to wound dressings based on synthetic polymers. These are classified as passive, interactive and bioactive products. Passive products are non-occlusive, such as gauze and tulle dressings, used to cover the wound to restore its function underneath. Interactive dressings are semi-occlusive or occlusive, available in the forms of films, foam, hydrogel and hydrocolloids. These dressings act as a barrier against penetration of bacteria to the wound environment

In another preferred embodiment of the product of the invention at least part of the support does not contain the immobilized SLS. Said part that does not contain the SLS may be required to adhere to a body part of a subject or to an object, or to place other agents required for the product function. Therefore, the determination of the part that does not contain SLS is determined by the skilled person in the field depending on the purpose of the required product, wherein said part can be as low as 0.1% or as high as 99.9% of the total area of the product, and can have whatever form is desired.

In another preferred embodiment of the product of the invention at least part of the region which does not contain the immobilized SPS comprises an adhesive compound that allows the adhesion of the haemostatic product to the skin.

The term "adhesion compound" as used herein refers to any substance that is capable of holding materials together in a functional manner by surface attachment that resists separation. In the present invention, said substance holds together the product of the invention and the skin of a subject. Said adhesion compound includes monomers, oligomers, polymers, and combinations thereof that may be used to bond at least two surfaces together temporarily or permanently, and/or may be used to bond to a surface. The term adhesive may also include monomers, oligomers, polymers, and combinations thereof in solution, hydrogel, suspension, and/or emulsion form, which upon drying, curing, or polymerization, may form an adhesive. The adhesive according to the present disclosure may be tacky to touch such as pressure sensitive adhesive. Adhesives described herein also include gel adhesives. Examples of adhesion compounds that can be used in the context of the present invention are, without any limitation, 2-Octyl cyanoacrylate, n-Butyl cyanoacrylate, ethyl 2-cyanoacrylate or any combination thereof.

The invention will be described by way of the following examples which are to be considered as merely illustrative and not limitative of the scope of the invention.

### EXAMPLES

### Example 1: Materials and Methods

### Example 1.0 - Materials used for SLS preparation

SPI, PROFAM 974, was supplied by ADM Protein Specialites Division (Netherlands). Sulphur content in SPI was determined using a Euro EA Elemental Analyzer. Moreover, amino acid composition of the SPI was determined with a Biochrom 30 + amino acid analyzer physiological system. CH was extracted from fresh squid pens (Loligo sp.), kindly supplied by a local fish market. First, we washed squid pens with water. Then, they were treated with NaOH (1 M) in a relation of 1:20 (w/v) at room temperature and under continuous stirring during 24 h in order to avoid the deacetylation of the native CH. After this process, we filtered the samples and the solid fraction (CH) was washed with distilled water until neutral pH. Finally, CH was freeze-dried and milled to obtain the powder. As determined by an elemental analysis the amount of C and N present in CH was, respectively, 42.1% and 6.2%. Besides, the average degree of acetylation was 95.9%. As for Glycerol (99.01% purity), it was provided by Panreac (Spain) and employed as a plasticizer.

### Example 1.1: Scanning Electron microscope (SEM) characterization

SEM micrographs were obtained by using a Hitachi S-4800 field emission scanning electron microscope (FE-SEM) (Hitachi High-Technologies Corporation, Tokyo, Japan) at a beam accelerated voltage of 5 kV. Materials were cut into discs of 8 mm diameter, mounted on a metal stub with adhesive tape and coated with gold under vacuum (JFC-1100) under an argon atmosphere prior to testing.

Pore sizes were determined measuring pore diameters from SEM micrographs using Image J software. Between 75 and 250 pores were measured for each material, which were randomly selected from different samples (n = 3 samples per group), and calculated the average pore size for each material.

To evaluate the porosity of the samples (15 - 20 mg, n = 3 samples per group), a liquid displacement method was performed using ethanol 98% as the liquid medium, due to its ability to permeate through scaffolds without inducing matrix swelling or shrinkage (G. Yang et al., 2018, Scientific Reports, 8(1), 1-13). Samples were immersed in a known volume (V1) of ethanol and degassed for 5 min with a vacuum pump. The total volume of ethanol and ethanol-impregnated sample was recorded as V2. Last, ethanol-impregnated samples were removed and the residual volume of ethanol was recorded as V3. The porosity ($\varepsilon$) of each sample was calculated using the following equation:

$$\varepsilon \, (\%) = \frac{V1 - V3}{V2 - V3} x100$$

### Example 1.2: Swelling capacity and liquid retention ratio under pressure (LRRP)

Swelling capacity of the materials (15 - 20 mg, n = 4 samples per group) was calculated immersing preweighed samples ($W_0$) of each material in 5 mL of PBS and weighing them again (Wt) at different time points - 5 s, 15 s, 30 s, 1 min, 5 min and 15 min - to obtain the swelling curves of each material. Swelling at each time point was calculated using the following equation:

$$Swelling\ (\%) = \frac{Wt - W0}{W0}x100$$

Liquid retention ratio under pressure (LRRP) was calculated in order to evaluate the ability of each material to retain the preabsorbed liquid when a certain pressure is applied. Samples of each material (20 - 25 mg, n = 6 samples per group) were immersed in PBS to absorb as much liquid as possible, and then they were weighed ($W_0$). Next, known weights - 10 g, 15 g, 30 g and 40 g - were serially placed upon the wet material, carefully collecting the released liquid and weighting the sample again. This procedure was repeated for each weight (WX). Then, LRRP was obtained using the following equation:

$$LRRP\ (\%) = \frac{Wx}{W0}x100$$

### Example 1.3: Hydrolytic degradation

Hydrolytic degradation was studied by immersing preweighed samples (20 - 25 mg, n = 4 samples per group) of each material in 2 mL PBS, and culturing them at 37 °C for different periods of time - 2, 5, 9 and 14 days -. Samples were removed from PBS, lyophilized and weighed in order to determine the remaining weight (%).

### Example 1.4: Mechanical characterization

The mechanical properties of the dry materials were tested by compression test and cyclic compression test (35 - 45 mg, n = 5 samples per group), using a mechanical tester - Instron 5969 equipped with a 100 N load cell - at a compressive rate of 0.5 mm s$^{-1}$ up to 70% strain. As MRC is commercialized in lyophilized form, it was expanded before the test, by wetting and completely drying it off. Several parameters, including relative stress, Young's modulus and damping coefficient, were calculated in order to compare the mechanical properties of the materials. Besides, an expansion force test was performed, hydrating samples of dry materials (35 - 45 mg, n = 5 samples per group) and recording the upwards stress displayed by the expansion of the material during water absorption.

### Example 1.5: Cytotoxic studies

Cytotoxicity of the materials was assessed according to the ISO 10993-5:2009 guidelines for biological evaluation of medical devices. Assays were performed both by direct contact with the cells - direct cytotoxicity - and by exposing the cells to a conditioned media of each material - indirect cytotoxicity -. In both assays viability of L-929 fibroblasts (ATCC® 30-2003™) was measured by employing CCK-8 reagent (Sigma-Aldrich, Spain). After incubating the cells with CCK-8 solution in medium (1 : 11) for 4 h, the absorbance was read with a plate reader (Infinite® 200 PRO series, Tecan Trading AG, Männedorf, Switzerland) at 450 nm, using 650 nm as the reference wavelength. Cells without material exposure were used as a control group (100% viability).

In the direct cytotoxicity assay, 35 000 cells were seeded per well in 500 μL/ well of EMEM complete medium in a 24-well plate. Next, the cells were incubated for 24 h at 37 °C. Afterwards, the medium was removed, 1 mL of fresh medium was added and each material (10 - 15 mg, n = 4 samples per group) was hydrated and placed in a transwell to bring it into direct contact with the bottom of the well. After 48 h of incubation, materials were removed, and the medium was replaced with 300 μL per well of CCK-8.

In the indirect cytotoxicity assay, L-929 fibroblasts were exposed to conditioned medium of each material (n = 5 samples per group), obtained incubating 100 mg of each material with 500 μL of EMEM at 37 °C for 24 h. Firstly, 5000 cells/well were seeded in a 96-well plate with 100 μL per well of medium and incubated for 24 h at 37 °C. After, the medium was replaced with 100 μL per well of the collected conditioned medium, and the plate was straightaway incubated at 37 °C for 24 h. Finally, conditioned medium was removed, and 110 μL per well of CCK-8 was added.

### Example 1.6: Blood swelling and degradation in blood

Maximum blood swelling capacity was tested by immersing preweighed ($W0$) samples (15 - 20 mg, n = 5 samples per group) of the materials in 500 μL of blood. After 2 minutes, to allow complete blood absorption, samples were weighed again (Wf). The maximum swelling capacity (%) was calculated using the following equation:

$$Swelling\ (\%) = \frac{Wf}{W0}x100$$

To evaluate the degradation of the materials in blood, preweighed ($W0$) samples (15 - 25 mg, n = 4 samples per group) of each material were placed in 24-well plates, using one plate for each time-point tested - 2, 4 and 7 days -. Blood was added to each sample in an amount of a 120% of its maximum blood swelling capacity, and the plates where incubated at 37 °C. At each time-point, samples were washed out with PBS, lyophilized and weighed (Wf). Degradation in blood was expressed as the remaining weight of the incubated and lyophilized samples with respect to their initial weight, according to the following equation:

$$Remaining\ weight\ (\%) = \frac{Wf}{W0}\,x100$$

## Example 1.7: Whole blood clotting *in vitro:* Hemoglobin quantification

Hemoglobin content of the samples was quantified using a Hemoglobin Assay Kit (Sigma-Aldrich, USA), by measuring absorbance of the samples (Is) with a plate reader (Infinite® 200 PRO series, Tecan Trading AG, Männedorf, Switzerland) at 400 nm.

For the whole blood clotting test, citrated whole blood - 9:1 whole blood to 3.8% sodium citrate - was collected from a healthy human donor. First, 0.2 mL of the citrated whole blood was added to preheated (30 min at 37 °C) samples of the materials disposed in a 24 well plate (25 - 35 mg, n = 5 samples per group). After this, 20 $\mu$L of CaCl$_2$ (0.2 M) were added to start coagulation. The plate with the materials was incubated under 30 rpm agitation for 10 min at 37 °C. Afterwards, 2 mL of deionized water were added in each well to hemolyze the RBCs that were not within the clot formed in the material. The deionized water containing the non-adhered and hemolyzed RBCs was collected and its hemoglobin content was quantified. 5 $\mu$L of CaCl$_2$ (0.2 M) and 50 $\mu$L of citrated whole blood were added to 750 $\mu$L of deionized water, and the absorbance of this solution was used as the reference value (Ir). The absorbance of an empty well was also measured (Io). Blood Clotting Index (BCI) of each sample was calculated using the following equation:

$$BCI\ (\%) = \frac{(Is - Io)}{(Ir - Io)}\,x100$$

With the purpose of visually evaluating the clotting capacity of each material, 150 mg of each material were disposed in Eppendorf tubes, subsequently adding 500 $\mu$L of citrated whole blood and 500 $\mu$L of CaCl$_2$ (10 mM). After incubating the Eppendorf tubes for 1 min at 37 °C, they were turned upside down and gently shaken to observe the formed clots.

## Example 1.8: Hemolysis assay *in vitro*

For the assessment of hemocompatibility *in vitro,* citrated whole blood - 9:1 whole blood to 3.8% sodium citrate - from a healthy donor was collected and diluted 1:5 in normal saline. Samples of each material (10 - 15 mg, n = 6 samples per group) were disposed in a 24-well plate. Diluted whole blood was added to each sample in an amount of the 80% of each material's swelling capacity. The plate was incubated for 1 h at 37 °C. Samples were moved to conical centrifuge tubes and centrifuged for 10 min at 3000 rpm. The supernatant of each sample was collected and its hemoglobin content was quantified as described in section Example 1.7. 150 $\mu$L of whole blood was added to deionized water and to normal saline, in order to use these solutions as positive (Ir) and negative (Io) controls, respectively. Hemolysis rate was calculated using the following equation:

$$Hemolysis\ (\%) = \frac{(Is - Io)}{(Ir - Io)}\,x100$$

## Example 1.9: Red blood cell adhesion *in vitro*

To evaluate red blood cell (RBC) adhesion to the material, citrated whole blood - 9:1 whole blood to 3.8% sodium citrate - was collected from a healthy donor. Samples of each material were cut (15 - 20 mg, n = 6 samples per group) and each sample was placed in a Petri dish. Citrated whole blood was added to each sample in an amount of the 80% of each material's swelling capacity, and they were incubated for 5 min at 37 °C. Next, 5-10 mL of deionized water were gently added by the edge of the dish until the water touched the sample and blood started to flow. More deionized water was added until a total volume of 50 mL for each sample. Each material was carefully moved to a clean Petri dish, and the liquid in each old Petri dish, - containing the RBCs that could not adhere to the sample -, was collected. The hemoglobin content of this solution was quantified as described in Example 1.7. This measurement accounts for *hemoglobin outside* the material, that is, the RBCs that could not adhere to the material.

Afterwards, *hemoglobin within* the materials was measured, which accounts for the amount of RBCs adhered to each sample. 10 mL of deionized water were added straightly on the material - so that the previously adhered RBCs would be released - and the hemoglobin content in the resultant solution was quantified as described in Example 1.7.

For both the *hemoglobin outside* and *within* the material, a positive control was prepared by mixing 200 $\mu$L of blood and 50 mL of deionized water. Hemoglobin concentration (mg dL$^{-1}$) of each sample was calculated using the following equation provided by the Hemoglobin Assay Kit, and results of each sample were normalized against the blood volume added to the control group:

$$[Hemoglobin] = \frac{(Is - Io)}{(Ir - Io)}\,x\,100\,\frac{mg}{dL}\,x\,df$$

where *Is* is the absorbance of the tested solution; *Io* is the absorbance of the blank (water); *Ir* is the absorbance of the calibrator provided in the Hemoglobin Assay Kit; *100 mg/dL* is the concentration of the diluted calibrator; *df* is the dilution factor, which was calculated for each sample depending on the volume of blood and deionized water added.

Citrated whole blood was also added to samples of each material (n = 3 samples per group) and the adhesion of RBCs was visually evaluated using SEM micrographs, obtained as described in Example 1.1.

### Example 1.10: Platelet adhesion *in vitro*

Adhesion of platelets to the materials was studied through a Lactate Dehydrogenase (LDH) Assay Kit (Sigma-Aldrich, USA), which allows to quantify platelet adhesion by measuring the LDH released by platelets when they are lysed, according to a reported method (No et al., 2002, International Journal of Food Microbiology, 74(1-2), 65-72). Briefly, citrated whole blood - 9:1 whole blood to 3.8% sodium citrate - was collected from a healthy donor and centrifuged at 480 g for 10 min at 4°C with 4 a/d, in order to obtain platelet-rich plasma (PRP). The serum above the buffy coat was collected from the centrifuged blood samples and kept in citrated tubes. Samples of each material (15 - 20 mg, n = 5 samples per group) were disposed in a 24-well plate and PRP was added to each sample in an amount of the 80% of each material's swelling capacity, and they were incubated for 30 min at 37 °C. Normal saline was gently added to each well until the samples were immersed, so that the non-adhered platelets could be removed from the material, and all samples were moved to a new 24-well plate. 1 mL of Triton X-100 1%-PBS was added straightly on the material, in order to lyse the platelets that had adhered to the material. After incubation for 1 h at 37 °C, each solution of Triton and lysed platelets was collected and LDH content was measured according to the protocol provided by the manufacturer (Sigma-Aldrich, USA) of the LDH Kit. To prepare the negative (Io) and positive (Ir) controls, 200 $\mu$L of PRP were added to 1 mL of PBS and Triton X-100 1%, respectively. Absorbances of the lysed platelets of each samples (Is) were measured with a plate reader (Infinite® 200 PRO series, Tecan Trading AG, Männedorf, Switzerland) at 400 nm, and normalized against the PRP volume added to the controls. LDH release (%) relative to the positive control was calculated using the following equation:

$$ LDH\ release\ (\%) = \frac{(Is - Io)}{(Ir - Io)} x 100 $$

PRP was also added to samples of each material (n = 3 samples per group) and the platelet adhesion was visually evaluated using SEM micrographs, obtained as described in Example 1.1.

### Example 1.11: *In vivo* hemostatic efficacy

To evaluate the hemostatic efficacy *in vivo,* a rat-tail amputation model was used. This experiment was conducted following the protocols approved by the Institutional Ethical Committee for Animal Experimentation of the University of the Basque Country. Wistar rats - with weights between 250 - 300 g (Janvier Labs, Le Genest-Saint-Isle, France) - were anesthetized with isoflurane (Isoflo®, Esteve, Spain), and 2.5 cm from the end of their tail was cut using a scalpel. The tail was placed in air for 10 s to ensure normal blood loss, and the wound was covered with the corresponding preweighed material (250 - 300 mg, n = 6 mice per group), holding it with a preweighed gauze (900 - 950 mg). The wound was uncovered every minute to check if bleeding persisted. For this reason, bleeding time (min) was assessed by assigning a score to each period at which bleeding ceased, as follows: 0 - 3 min : 1; 3 - 6 min : 2; 6 - 9 min : 3; >9 min : 4. Therefore, a lower bleeding time score represents a faster hemostatic effect. The blood-impregnated samples were weighed again to calculate total blood loss (g).

### Example 1.12: Statistical Analysis

Results were expressed as the mean $\pm$ standard deviation. When normally distributed, results were analyzed through a one-way ANOVA test. Bonferroni or Tamhane post-hoc analysis was applied based on the Levene test for the homogeneity of variances. In reverse, non-normally distributed data was analyzed by Mann-Whitney's nonparametric analysis. Sample size of each experiment is indicated in the corresponding materials and methods section. p < 0.05 was considered statistically significant. Moreover, all the statistical computations were performed using SPSS 25.0 (SPSS®, Inc., Chicago, IL, USA).

### Example 2: Results

### Example 2.0: Preparation of the SLS of the invention

The SLS of the invention was prepared based on a green strategy using valorized by-products of the food industry (Las Heras et al., 2020, Green Chem., 22, 3445-3460).

### Example 2.1: Pore size and porosity

Pore structure of biomaterials that aim to be used as wound healing agents - either to treat hemorrhages or other types of wounds - is of great importance. In this experiment, the gauze was the material with the highest pore size - 479 $\pm$ 252 $\mu$m -, while MRC and SP-CH showed a very similar pore size distribution, 144 $\pm$ 63 and 141 $\pm$ 124 $\mu$m, respectively (Figure 1A). MRC and SP-CH showed no significant difference in mean pore size (p > 0.05), while both of them showed significant differences with respect to gauze (p < 0.001).

In this context, MRC and SP-CH showed similar porosity (Figure 1B), 92.97 ± 4.39% and 86.95 ± 1.58%, respectively. Meanwhile, the gauze showed significantly higher porosity than SP-CH, 96.68 ± 0.12%. On the other hand, SEM micrographs (Figure 1C) clearly show that internal structures of the three materials differ. The gauze shows thousands of intertwined fibers forming a mesh, but the space between the fibers does not present well defined pores, which are required for good liquid retention. For their part, MRC and SP-CH show an internal microstructure of well- defined interconnected pores, which in the case of MRC are more circular and in the case of SP-CH present a more amorphous structure. Together with a similar pore size and porosity, this makes MRC and SP-CH very similar regarding to their internal microarchitecture, which completely differs from that of the gauze.

**Example 2.2: Swelling capacity and Liquid Retention Ration under Pressure (LRRP)**

The capacity of biomaterials to absorb fluids is important as it will determine their abilities to absorb the blood present in hemorrhages. We tested the water swelling profiles of the three materials by measuring swelling at different time points (Figure 1D). The three materials showed similar water uptake capacity, with the maximum swelling capacity ranging between 1,334 ± 34.2% (MRC) and 1,442 ± 266.91% (SP-CH) of its initial weight after 15 minutes. During that period of time, the gauze absorbed 1412 ± 181.6% of its initial weight. Even if the maximum absorption capacity was similar for the three materials, the swelling profile turned out to be quite different. While SP-CH and gauze achieved a 1,383 ± 167.78% and 1,324 ± 302.31% of their initial weight within only 5 seconds, respectively, MRC could only swell a 178 ± 21.36% of its initial weight during the same time. This shows that even if all of them displayed similar maximum absorption capacity, MRC showed delayed swelling, only equaling SP-CH and gauze after 60 seconds. The water absorption property appears to be especially important in the treatment of epistaxis as the principal way in which nasal packs arrest bleeding is by exerting pressure on the damaged blood vessels of the nasal mucosa to promote hemostasis. After being inserted in the nostril, the nasal pack absorbs wound fluids and expands, maintaining pressure on the wound site. This pressure should be high enough to control the bleeding, but not as high to damage the nasal cavity. Complications associated with excessive pressure include movement of the pack from its initial position, obstructed breathing and reduced sense of smell, and even necrosis of mucosa and cartilage.

Nevertheless, when the pack puts pressure on the nostril's walls, the nostril's walls also put pressure on the pack, thus desorbing part of the previously absorbed fluids. For this reason, we evaluated the capacity of the materials to retain the absorbed liquid under pressure. In this experiment (Figure 1E) both MRC and SP-CH showed significantly greater (p < 0.001) water retention capacity compared to gauze, independently of the pressure applied. Besides, MRC displayed significantly better retention at low pressures compared to SP-CH, although both materials turned out to similar (p > 0.05) retaining values for pre-absorbed water when the maximum weight - 40 gr - was applied, with MRC maintaining 49.58 ± 4.37% and SP-CH 45.83 ± 4.54% of their maximum swelling capacity, while gauze maintained just 34.44 ± 2.35%. Therefore, even if the three materials showed similar water swelling capacity, gauze is not able to effectively retain the absorbed water when a pressure is applied, and thus loses its ability to exert pressure to the bleeding site, likely due to its internal microstructure.

**Example 2.3: Hydrolytic degradation**

Degradability is a property of increasing popularity among biomaterials for nasal packings, as it reduces both patient discomfort and rebleeding upon removal, among other advantages. In this vein, both gauze and MRC showed no degradation during the 14 days of experiment (Figure 1F), while SP-CH degraded 25.96 ± 3.22% of its initial weight after 48h. After that measurement, SP-CH showed no more degradation on the following days (p > 0.05). These results clearly show that both gauze and MRC are totally non-absorbable nasal packs, while SP-CH can lose about a 25% of its initial weight after 48 hours via hydrolytic degradation. This may probably be enough to relieve patient's discomfort and damage associated with pack removal, and to avoid rebleeding episodes, which would suppose a great advantage both for the patient and the clinician (Wormald et al., 2006, American Journal of Rhinology, 20(1), 7-10).

**Example 2.3: Mechanical characterization**

For appropriate handling and clinical use nasal packs should present good mechanical properties and resistance to deformation, thus alleviating patient's concerns about discomfort during the treatment and providing mechanical stability.

We carried out a cyclic compression stress-strain test with 10 cycles (Figure 2A) to characterize the mechanical properties of the materials (Figure 2B-D). These curves show an initial plateau region, followed by a hyperelastic region at higher strains, which is more noteworthy for the gauze and SP-CH than for MRC. For the three materials the relationship between stress and strain is nonlinear, exhibiting nonlinear viscoelasticity, similarly to many body tissues and extracellular matrix components. This viscoelasticity could be confirmed by the loading and unloading curves of each cycle taking different paths, which occurs due to energy dissipation during deformation of the material (Z. Wang et al., 2016, Viscoelastic and Viscoplastic Materials, 2, 64), producing an hysteresis loop between loading and unloading curves. The area under the loading curve represents the total input energy of the cycle and the area under the unloading curve represents the elastic strain energy, while the area between the two curves accounts for the dissipated energy during the compression cycle (D. Yang et al., 2020, Alexandria Engineering Journal, 59(5), 3587-3597). In addition to this, gauze showed the highest stress at a strain of 70%, both for cycle 1 and for cycle 10, while MRC showed the lowest, meaning that gauze was the most difficult material to deform up to a strain of 70%, and MRC the easiest one, which could be due to the internal microstructure of each one. SP-CH required an intermediate stress to be deformed up to a 70% of strain.

Nevertheless, the cyclic compression test revealed that the stress needed to deform the materials decreased substantially cycle after cycle (Figure 2E), losing their maximum ability to resist deformation. This phenomenon, which is defined as cyclic softening (Jürgens et al., 2019, Metals, 9(1)), is probably a consequence of irreversible deformations of the internal microarchitecture caused by the compression (L. Zhu et al., 2017, RSC Advances, 7(69), 43755-43763). Results of the relative maximum stress (%) (Figure E) show that the gauze could only maintain an $80.2 \pm 2.04\%$ of its maximum stress after 10 cycles, compared to the maximum stress of cycle 1. This is significantly less ($p<0.01$) than MRC and SP-CH, that displayed a relative stress of $87.34 \pm 5.24\%$ and $88.63 \pm 1.63\%$ after 10 cycles, respectively.

Young's modulus defines the ability of a material to withstand changes in length when subjected to compressive loads, and it is useful to measure the stiffness of a material (L. Wang et al., 2003, Journal of Applied Oral Science, 11(3), 162-167): the higher the Young's modulus, the higher the stiffness of the material. Since the three materials present a nonlinear behavior, Young modulus in not constant but changes depending on the position in the stress-strain curve, and is defined by the slope of the tangent to the curve. Thus, we calculated the Young's modulus for all the strains of the first cycle of each biomaterial (Figure 2F). Initially, the three samples presented a high Young's modulus, which rapidly decreased when strain started to increase. As strained became higher, the Young modulus of gauze and SP-CH started to increase again at strains coinciding with the ones in which the hyperelastic region of each material began - around 25% of strain for the gauze and 50% of strain for SP-CH -. This could indicate that at 25% of strain, all the cotton layers forming the gauze are very compact, and consequently the stiffness of the sample strongly increases. Meanwhile, the internal microstructure of the SP-CH would not get completely compact until a strain of 50%. MRC showed no meaningful increase of its Young's modulus at higher strains, indicating that its stiffness remained low throughout all the compression process. Summing up, while gauze becomes rigid at low strains, both MRC and SP-CH remain flexible until a strain of 50% is achieved.

Since a nasal pack is inevitably subjected to mechanical compression when inserted in the nostril, we evaluated the damping coefficient of the three materials for cycle 1 and 10, calculating it as the ratio of the energy dissipation - area of the hysteresis loop - and the total input energy - area under de loading curve - for the given cycle (Orban, 2011, In Journal of Physics: Conference Series [Vol. 268, Issue 1], IOP Publishing). For the first cycle, SP-CH presented a damping coefficient of $0.72 \pm 0.02$, which was significantly higher ($p<0.001$) than the ones obtained for gauze and MRC, $0.59 \pm 0.05$ and $0.29 \pm 0.01$, respectively. Comparing the first cycle's coefficient to the cycle 10's, all the materials significantly ($p<0.001$) lost damping capacity, probably due to irreversible alterations in their internal microstructure caused by the cyclic compression. Even though, SP-CH presented a damping coefficient of $0.57 \pm 0.03$ in cycle 10, which is still significantly higher ($p<0.001$) than the ones calculated for gauze and MRC, $0.38 \pm 0.03$ and $0.21 \pm 0.01$, respectively. These results indicate that our material has an increased capacity to dampen the impact waves and mechanical forces that may be produced with the pack placed in the nasal cavity.

To evaluate the upwards force displayed by the materials when they absorb water, we performed an expansion force test, as illustrated in Figure 2H. This force is representative of the force these materials would display against the nasal cavity's walls when absorbing fluids within the nostril, and therefore this property seems to be of special interest for a nasal pack. Figure 2I shows the relative expansion strength curves of the materials throughout time, compared to their initial strength. Both gauze and MRC displayed null expansion force when absorbing water, even decreasing their initial strength after adding water to the sample. In contrast, SP-CH's relative expansion curve evinces that when absorbing water it displays an increasing upwards force, caused by its expansion. This is numerically represented in Figure 2J, in which the maximum expansion force (%) displayed during the experiment is calculated with respect to the initial expansion force. Results confirmed that gauze and MRC have no capacity to display an upwards force when absorbing water, just increasing the initial expansion force up to a $106.64 \pm 10.66\%$ and $109.88 \pm 16.97\%$, respectively. Conversely, SP- CH presented an expansion force of $249.59 \pm 24.3\%$ with respect to the start of the experiment, which supposes a significantly higher ($p<0.001$) expansion force than for gauze and MRC. Thus, it would probably be able to exert a higher pressure to the bleeding site when absorbing wound fluids within the nostril to promote hemostasis.

## Example 2.4: Cytotoxicity studies

We performed direct and indirect cytotoxicity assays to confirm the biocompatibility of the materials. We carried out these assays following an adapted protocol from the ISO 10993 5:2009 guidelines for biological evaluation of biomedical devices. Both in direct (Figure 3A) and indirect (Figure 3B) cytotoxicity assays the three materials showed cell viability above 70%, and thus their biocompatibility could be confirmed. In both assays, no significant differences were found in biocompatibility between MRC and SP-CH (p > 0.05), but gauze showed significantly higher biocompatibility in comparison to SP-CH in the direct cytotoxicity assay (p < 0.05). In contrast, in the indirect cytotoxicity assay, SP-CH turned out to be significantly more biocompatible than the gauze (p < 0.05).

We also performed the Live/Dead staining method to visually prove that these biomaterials allowed viability of L-929 cells cultured upon them (Figure 3C), with practically all cells alive (green colored) in all the samples, and just a few dead cells - red colored - present in the MRC images. These studies proved that the SP-CH SLS is adequate for biomedical use without causing any cytotoxicity to the cells present in the nasal cavity and surrounding tissues.

**Example 2.5: Degradation of blood**

To test the degradation profile of the materials in a more biologically relevant fluid, we evaluated their blood degradation profile (Figure 4A). Results showed that SP-CH degrades in contact with blood, while gauze and MRC do not, as confirmed by the images of the materials after incubation with blood (Figure 4B). After 4 days of incubation in blood, the gauze presented a weight of 110.07 ± 1.89% with respect to its initial weight, while MRC presented a remaining weight of 102.93 ± 1.23%. In contrast, SP-CH maintained a 79.3 ± 1.79% of its weight after 4 days of incubation in blood, meaning that it could degrade a 20.7 ± 1.79% of its initial weight during that period of time. This would probably facilitate its removal from the patient's nostril once the nasal pack reaches its goal.

Interestingly, the gauze and MRC not only did not degrade but their weight slightly increased after being incubated with blood, although samples where lyophilized before weighing, to eliminate all the fluids present in the samples. This might be due to blood cells and proteins adhering to these materials, which could not be completely eliminated. This indicates that these materials could have degraded to some level, but that the adherence of these blood components would balance out that degradation. Therefore, the degradation profile of SP-CH could also have been underestimated by the adhesion of blood components as in the case of gauze and MRC, which would mean that our material has even better blood degradation properties. The fact that SP-CH degrades a fifth part of its weight after 4 days, confirms that this material can be used as a partly absorbable nasal pack, with the benefits that this entails, thereby alleviating concerns such as pain during removal and wound healing disruption, or decreasing the incidence of nasal adhesions.

**Example 2.6: Blood swelling capacity**

Blood swelling capacity is essential for biomaterials intended to be used in hemorrhagic wounds, and seems to be of greater importance than the water swelling profile because of the presence of blood in the site of application. For this reason, we evaluated the maximum blood absorption of the three materials (Figure 4C). Gauze showed a blood swelling capacity of 1,091.13 ± 20% with respect to its initial weight. MRC absorbed significantly more blood than the gauze (p < 0.05), with a mean blood absorption of 1,276.1 ± 40.08%. For its part, SP-CH showed significantly higher blood swelling capacity compared to both gauze and MRC (p < 0.05), absorbing up to 1,442.87 ± 116.76% of blood respect to its initial weight. This increased blood swelling capacity of SP-CH is advantageous from different point of views. On the one hand, higher volumes of blood absorbed would result in a stronger tamponade effect to the injury, which is necessary to arrest bleeding. On the other hand, the more blood volume the pack absorbs the higher amount of RBCs and platelets will be concentrated within the biomaterial, promoting hemostasis and thrombus formation.

**Example 2.7: Hemolysis assay *in vitro***

We conducted a hemolysis assay in order to assess the hemocompatibility of the materials, as biomaterials in contact with blood are required to induce minimal hemolysis, which is defined as the destruction of RBCs due to shear stress or changes in osmotic pressure (Weber et al., 2018, Frontiers in Bioengineering and Biotechnology, 6(July)). In this context, the gauze showed a hemolysis rate of 3.27 ± 0.62%, while MRC showed significantly decreased hemolysis rate in comparison with both gauze and SP-CH (p < 0.05), exactly 2.06 ± 0.7%. SP-CH caused a hemolysis rate of 3.54 ± 0.81%, which supposes no significant difference in hemocompatibility with gauze (p > 0.05). Since 5% of hemolysis is the maximum permitted for biomaterials (J. Zhu et al., 2019, Chemistry of Materials, 31(12), 4436-4450), the three tested biomaterials were proved to be non-hemolytic, and the hemocompatibility of SP-CH could be confirmed.

**Example 2.8: Blood clotting *in vitro***

The ability to induce the clotting of blood is of huge importance for biomaterials intended to treat bleeding wounds such as nasal hemorrhages, and thus we assessed blood clotting *in vitro* for the three materials. For this, we calculated the Blood Clotting Index (BCI%) of the materials (Figure 4E), with a lower blood-clotting index indicating a faster blood-clotting rate (Chen et al., 2020, Biomaterials Science, 8(13), 3760-3771). MRC showed the highest BCI, and thus the slowest blood-clotting rate, with an index of 82.7 ± 4.52%. The gauze and SP-CH showed significantly lower (p < 0.001) BCI compared to MRC, 37.7 ± 18.2% and 34.42 ± 5.91% respectively. With the aim of visually assessing the *in vitro* blood clotting capacity of the samples, we performed the tube inversion method (Figure 4F). This assay reinforced the results obtained in Figure 4E, confirming the excellent blood clotting capacity of SP-CH, as the blood added to the tubes remained in the upper part forming a strong clot because of the interaction with the SP-CH sponge. For their part, the gauze and MRC were not able to form clots that maintained all the blood in the upper part of the tube, with the majority of the added blood falling to the bottom of the Eppendorf, and thus confirming their decreased hemostatic effect compared to that of SP-CH.

### Example 2.9: RBC ADHESION *IN VITRO*

Adhesion of RBCs to the surface of the biomaterial is of great importance to promote hemostasis by the formation of a stable and strong blood plug. The measurement of hemoglobin outside the materials (Figure 5A) represents the amount of RBCs that could not adhere to the material, and thus, a lower hemoglobin concentration outside the material indicates a higher adhesion of RBCs. In this vein, both gauze and MRC showed no significant difference (p > 0.05) with respect to a control in which no adhesion of RBCs was produced, showing hemoglobin concentrations of 15,535 ± 1,977 mg/dL and 15,882 ± 2,231 mg/dL, respectively, while in the control group we measured a hemoglobin concentration of 16,245 ± 2,365 mg/dL. SP-CH showed significantly decreased (p <0.001) hemoglobin concentration compared to the other groups, concretely 10,346 ± 1,905 mg/dL, indicating higher RBC adhesion to the surface of the biomaterial. Figure 5B shows the appearance of the materials throughout the experiment, and a higher hemoglobin concentration - and consequently a higher adhesion of RBCs to SP-CH - can be visually confirmed.

In addition to this, we assessed the hemoglobin concentration within the materials (Figure 5C), which can be used as an indicator of the amount of RBCs adhered to the surface of the biomaterials, and thus, in this assay, a higher hemoglobin concentration indicates an increased level of RBC adhesion. Results of this experiment confirmed the outstanding RBC adhesion to SP-CH compared to gauze and MRC. While our biomaterial presented a hemoglobin concentration of 6,661 ± 571 mg/dL, these concentrations were 1,801 ± 133 mg/dL and 2,004 ± 451 mg/dL in the case of the gauze and MRC, respectively. Again, the hemoglobin concentrations - and consequently the RBC adhesion presented graphically - can be visually confirmed in the images of the biomaterials during the experiment (Figure 5D). The SP-CH's sample looked darker due to a higher RBC adhesion, and the finally collected and quantified liquid presented a redder color because of an elevated hemoglobin concentration.

Lastly, we evaluated the RBC adhesion through SEM micrographs (Figure 5E) of samples of each biomaterial, which had been previously incubated with blood. Even if MRC shows an acceptable amount of RBCs adhered, the surface of SP-CH shows increased concentration of RBCs, forming big aggregates of cells which are adhered both to each other and to the surface of our biomaterial. Conversely, RBC adhesion to gauze is minimal compared to the other materials.

These results elucidate the hypothetical hemostatic mechanism of SP-CH, as it has a great capacity to absorb blood and bind the RBCs, concentrating them at its surface. In this sense, the role of RBCs in hemostasis is gaining interest, and recent studies indicate that RBCs migrate to the bleeding site and push platelets toward the endothelium - margination - in a hematocrit- and shear-dependent manner.

### Example 2.10: Platelet adhesion *in vitro*

We evaluated the platelet adhesion of the three materials using an indirect method (Figure 6A) that measures the amount of LDH released from a sample, which is directly proportional to the amount of platelets within it. Both gauze and MRC showed decreased platelet adhesion, with a LDH release of just a 23.46 ± 3.75% and 24.86 ± 3.61%, respectively, of the one measured for a positive control in which all the added platelets were lysed. For its part, SP-CH presented a LDH release of a 47.81 ± 8.4% of that measured for the positive control, which means a significantly higher (p < 0.001) platelet adhesion compared to both the gauze and MRC. SEM micrographs of the materials cultured with PRP (Figure 6B) confirmed the quantitative results obtained in the *in vitro* platelet adhesion assay, showing that platelet adhesion to gauze and MRC is minimal, while SP-CH shows bigger amounts of platelets adhered to its surface, forming aggregates of platelets, with even some of them activated.

### Example 2.11: *In vivo* hemostatic efficacy

A rat-tail amputation model was used to evaluate the *in vivo* hemostatic properties of the three materials. The gauze obtained a mean bleeding time score of 2.8 ± 0.84, which was similar to MRC's, 2.5 ± 1.05. Meanwhile, SP-CH obtained a significantly lower (p > 0.05) bleeding time score, 1.8 ± 0.45, meaning that it could promote hemostasis *in vivo* faster than both gauze and MRC (Figure 7A). Regarding to blood loss, no significant differences were found between groups. The gauze presented a mean blood loss of 1.08 ± 1.01 g, MRC 1.17 ± 0.84 g and SP-CH 0.61 ± 0.35 g (Figure 7B). However, it is noteworthy that both gauze and MRC showed an increased deviation among replicates, while blood loss in the SP-CH group remained consistent in all the tested samples, guaranteeing a homogeneous hemostatic efficacy.

Nevertheless, the bleeding time results support those obtained in the *in vitro* experiments, where SP-CH showed superior hemostatic properties compared to the gauze and MRC.

**Claims**

1. A sponge-like scaffold comprising soy protein isolate (SPI), chitin (CH) and a plasticizer for use in promoting haemostasis.

2. The sponge-like scaffold for use according to claim 1, wherein the scaffold has a porosity of around between 70% and 80%, and a pore size of around between 0.5 mm and 1.5 mm.

3. The sponge-like scaffold for use according to any previous claim wherein the content of CH is of about 30% (w/w) with respect to the dry SPI content.

4. The scaffold for use according to any of the previous claims, wherein the CH has an acetylation degree of at least 75%.

5. The scaffold for use according to claim 4, wherein the CH has an acetylation degree of at least 97%.

6. The scaffold for use according to any of the previous claims, wherein the CH contains β-chitin.

7. The scaffold for use according to any of the previous claims, where in the SPI comprises Glu, Asp, Leu, Ser, Gly, Ala and Pro, in a percentage with respect to the total amino acid residues present in the SPI, of at least 6% each.

8. The scaffold for use according to any one of the previous claims, wherein the SPI has a sulphur content of around less than 2%, preferably less than 1%.

9. The scaffold for use according to any of claims 1 to 8 wherein the plasticizer is a polyol, preferably glycerol.

10. The scaffold for use according to claim 9 wherein the plasticizer content is of about 30% (w/w) with respect to the SPI content.

11. The scaffold for use according to any one of the previous claims wherein the promotion of haemostasis is in the treatment of a bleeding wound.

12. The scaffold for use according to claim 11, wherein the bleeding wound is a haemorrhage.

13. An haemostatic product that comprises a sponge-like scaffold as defined in any of claims 1 to 12 and a support wherein the sponge-like scaffold is immobilized in a support.

14. The haemostatic product according to claim 13, wherein the support is a gauze, a tissue, a plastic tissue, a stick or a string.

15. The haemostatic product according to claim 13 or 14, wherein at least part of the support does not contain the immobilized sponge-like scaffold and at least a region within the part of the support which does not contain the immobilized sponge-like scaffold comprises an adhesive compound that allows the adhesion of the haemostatic product to the skin.

**Patentansprüche**

1. Ein schwammartiges Gerüst, umfassend Sojaproteinisolat (SPI), Chitin (CH) und einen Weichmacher zur Verwendung bei der Förderung der Hämostase.

2. Das schwammartige Gerüst zur Verwendung gemäß Anspruch 1, wobei das Gerüst eine Porosität von etwa 70 % bis 80 % und eine Porengröße von etwa 0,5 mm bis 1,5 mm aufweist.

3. Das schwammartige Gerüst zur Verwendung gemäß einem der vorherigen Ansprüche, wobei der Gehalt an CH etwa 30 % (Gew./Gew.) in Bezug auf den Trocken-SPI-Gehalt beträgt.

4. Das Gerüst zur Verwendung gemäß einem der vorherigen Ansprüche, wobei das CH einen Acetylierungsgrad von mindestens 75 % aufweist.

5. Das Gerüst zur Verwendung gemäß Anspruch 4, wobei das CH einen Acetylierungsgrad von mindestens 97 % aufweist.

6. Das Gerüst zur Verwendung gemäß einem der vorhergehenden Ansprüche, wobei das CH β-Chitin enthält.

7. Das Gerüst zur Verwendung gemäß einem der vorhergehenden Ansprüche, wobei das SPI Glu, Asp, Leu, Ser, Gly, Ala und Pro in einem prozentualen Anteil von jeweils mindestens 6 % bezogen auf die Gesamtmenge der im SPI vorhandenen Aminosäurereste umfasst.

8. Das Gerüst zur Verwendung gemäß einem der vorhergehenden Ansprüche, wobei das SPI einen Schwefelgehalt von weniger als etwa 2 %, vorzugsweise weniger als 1 %, aufweist.

9. Das Gerüst zur Verwendung gemäß einem der Ansprüche 1 bis 8, wobei der Weichmacher ein Polyol, vorzugsweise Glycerin, ist.

**10.** Das Gerüst zur Verwendung gemäß Anspruch 9, wobei der Weichmachergehalt etwa 30 % (Gew./Gew.) in Bezug auf den SPI-Gehalt beträgt.

**11.** Das Gerüst zur Verwendung gemäß einem der vorhergehenden Ansprüche, wobei die Förderung der Hämostase bei der Behandlung einer blutenden Wunde erfolgt.

**12.** Das Gerüst zur Verwendung gemäß Anspruch 11, wobei die blutende Wunde eine Hämorrhagie ist.

**13.** Ein hämostatisches Produkt, das ein schwammartiges Gerüst gemäß einem der Ansprüche 1 bis 12 und einen Träger umfasst, wobei das schwammartige Gerüst in einem Träger immobilisiert ist.

**14.** Das hämostatische Produkt gemäß Anspruch 13, wobei der Träger eine Gaze, ein Gewebe, ein Kunststoffgewebe, ein Stäbchen oder eine Schnur ist.

**15.** Das hämostatische Produkt gemäß Anspruch 13 oder 14, wobei mindestens ein Teil des Trägers das immobilisierte schwammartige Gerüst nicht enthält und mindestens ein Bereich innerhalb des Teils des Trägers, der das immobilisierte schwammartige Gerüst nicht enthält, eine Klebstoffverbindung umfasst, die die Haftung des hämostatischen Produkts an der Haut ermöglicht.

**Revendications**

**1.** Un échafaudage spongieux comprenant de l'isolat de protéines de soja (SPI), de la chitine (CH) et un plastifiant, destiné à être utilisé pour favoriser l'hémostase.

**2.** L'échafaudage spongieux pour utilisation selon la revendication 1, dans lequel l'échafaudage a une porosité comprise entre environ 70 % et 80 %, et une taille de pore comprise entre environ 0,5 mm et 1,5 mm.

**3.** L'échafaudage spongieux pour utilisation selon l'une quelconque des revendications précédentes, dans lequel la teneur en CH est d'environ 30 % (p/p) par rapport à la teneur en SPI sec.

**4.** L'échafaudage pour utilisation selon l'une quelconque des revendications précédentes, dans lequel la CH a un degré d'acétylation d'au moins 75 %.

**5.** L'échafaudage pour utilisation selon la revendication 4, dans lequel la CH a un degré d'acétylation d'au moins 97 %.

**6.** L'échafaudage pour utilisation selon l'une quelconque des revendications précédentes, dans lequel la CH contient de la β-chitine.

**7.** L'échafaudage pour utilisation selon l'une quelconque des revendications précédentes, dans lequel le SPI comprend Glu, Asp, Leu, Ser, Gly, Ala et Pro, dans un pourcentage par rapport aux résidus d'acides aminés totaux présents dans le SPI, d'au moins 6 % chacun.

**8.** L'échafaudage pour utilisation selon l'une quelconque des revendications précédentes, dans lequel le SPI a une teneur en soufre d'environ moins de 2 %, de préférence moins de 1 %.

**9.** L'échafaudage pour utilisation selon l'une quelconque des revendications 1 à 8, dans lequel le plastifiant est un polyol, de préférence du glycérol.

**10.** L'échafaudage pour utilisation selon la revendication 9, dans lequel la teneur en plastifiant est d'environ 30 % (p/p) par rapport à la teneur en SPI.

**11.** L'échafaudage pour utilisation selon l'une quelconque des revendications précédentes, dans lequel la promotion de l'hémostase a lieu pendant le traitement d'une plaie saignante.

**12.** L'échafaudage pour utilisation selon la revendication 11, dans lequel la plaie saignante est une hémorragie.

**13.** Un produit hémostatique qui comprend un échafaudage spongieux selon l'une quelconque des revendications 1 à 12 et un support, dans lequel l'échafaudage spongieux est immobilisé dans un support.

**14.** Le produit hémostatique selon la revendication 13, dans lequel le support est une gaze, un tissu, un tissu en plastique, un bâtonnet ou une ficelle.

**15.** Le produit hémostatique selon la revendication 13 ou 14, dans lequel au moins une partie du support ne contient pas l'échafaudage spongieux immobilisé et au moins une zone située dans la partie du support qui ne contient pas l'échafaudage spongieux immobilisé comprend un composé adhésif qui permet l'adhérence du produit hémostatique à la peau.

Fig. 1

Fig. 1 (continuation)

# Fig. 1 (continuation)

**Fig. 2**

Fig. 2 (continuation)

**Fig. 2 (continuation)**

**Fig. 2 (continuation)**

Fig. 2 (continuation)

EP 4 401 799 B1

Fig. 3

29

Fig. 4

Fig. 5

Fig. 5 (continuation)

Fig. 5 (continuation)

**Fig. 6**

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017015488 A1

**Non-patent literature cited in the description**

- **LU et al.** *Biomacromolecules*, 2004, vol. 5, 1046
- Carbohydrate Polymers, Polymers. **SONG et al.** Applied Science Publishers. LTD Barking, 2021, vol. 73
- Effects of degree of deacetylation on haemostatic performance of partially deacetylated chitin sponges. **SONG XIAOQIANG et al.** CARBOHYDRATE POLYMERS, APPLIED SCIENCE PUBLISHERS. LTD BARKING, 28 August 2021, vol. 273
- **G. YANG et al.** *Scientific Reports*, 2018, vol. 8 (1), 1-13
- **NO et al.** *International Journal of Food Microbiology*, 2002, vol. 74 (1-2), 65-72
- **LAS HERAS et al.** *Green Chem.*, 2020, vol. 22, 3445-3460
- **WORMALD et al.** *American Journal of Rhinology*, 2006, vol. 20 (1), 7-10
- **Z. WANG et al.** *Viscoelastic and Viscoplastic Materials*, 2016, vol. 2, 64
- **D. YANG et al.** *Alexandria Engineering Journal*, 2020, vol. 59 (5), 3587-3597
- **JÜRGENS et al.** *Metals*, 2019, vol. 9 (1)
- **L. ZHU et al.** *RSC Advances*, 2017, vol. 7 (69), 43755-43763
- **L. WANG et al.** *Journal of Applied Oral Science*, 2003, vol. 11 (3), 162-167
- **ORBAN**. In Journal of Physics: Conference Series. IOP Publishing, 2011, vol. 268
- **WEBER et al.** *Frontiers in Bioengineering and Biotechnology*, 2018, vol. 6
- **J. ZHU et al.** *Chemistry of Materials*, 2019, vol. 31 (12), 4436-4450
- **CHEN et al.** *Biomaterials Science*, 2020, vol. 8 (13), 3760-3771